# EUROPEAN PATENT APPLICATION

(11) **EP 2 685 257 A1**
(43) Date of publication of application: **15.01.2014**
(21) Application number: 12755365.9
(22) Date of filing: 07.03.2012
(51) Int. Cl.: G01N 30/88, G01N 30/04, G01N 30/06, G01N 30/72, G01N 30/86

(54) **ANALYSIS METHOD**

(30) Priority: 08.03.2011 JP 2011049726
(71) Applicant: Sanwa Kagaku Kenkyusho Co., Ltd, Nagoya-shi, Aichi 461-8631 (JP)
(72) Inventor: MIYACHI, Atsushi, Nagoya-shi Aichi 461-8631 (JP); YASUDA, Yoshika, Nagoya-shi Aichi 461-8631 (JP); HIROOKA, Hiroko, Nagoya-shi Aichi 461-8631 (JP)
(74) Representative: Lenthall, Joseph
(86) International application number: PCT/JP2012/055873
(87) International publication number: WO 2012/121302

(57) **Abstract**

**Solution to problem**

The present invention provides an analysis method for a glucagon-secretin family peptide in a sample, the method comprising:
a cleavage step of a peptide bond of aspartic acid of a glucagon-secretin family peptide in a sample to obtain peptide fragment;
a separation/purification step of separating and purifying the peptide fragments cleaved in the cleavage step using liquid chromatography to elect a peptide fragment, a substance to be measured, on an N-terminal glucagon-secretin family peptide; and
an analysis step of carrying out mass spectrometry to the peptide fragments separated and purified in the separation/purification step to detect the peptide fragment on the N-terminal glucagon-secretin family peptide.

## Description

### Technical Field

The present invention relates to an analysis method for glucagon-secretin family peptides and a quantitative method for glucagon-secretin family peptides in a sample using such analysis method.

### Background Art

The glucagon-secretin family peptide is a generic name for peptides which have a high amino acid sequence homology to glucagon and the like. An accurate understanding of the concentration of glucagon-secretin family peptide in e.g. a living body is important in terms of function studies, an understanding of disease status of patients or the development of drugs for improving the diseases.

As quantitative methods for glucagon-secretin family peptides, quantitative methods using antibodies are general as typified by e.g. enzyme-linked immunosorbent assay (ELISA) and radioimmunoassay (RIA). To prepare antibodies, however, a long period of time and high expenses are required. Even when an antibody is obtained, the problem of cross-reactivity can occur, in which the antibody also reacts to other physiologically active substances which have a high homology to the peptides.

Quantitative methods for glucagon-secretin family peptides using mass spectrometry (MS) have been reported in recent years (Non-patent Documents 1 to 3, Patent Document 1). Non-patent Documents 1 and 2, however, use antibodies as pretreatment, and Patent Document 1 and Non-patent Document 3 are not yet satisfying in terms of quantitative sensitivity and the like.

### Citation List

### Patent Literature

Patent Document 1: International Publication No. WO 2008/154619 A

### Non Patent Literature

Non-patent Document 1 J Chromatogr A, 2001, 926, 21
Non-patent Document 2 J Chromatogr B, 2004, 803, 91
Non-patent Document 3 J Proteome Res, 2009, 8, 3487

### Summary of Invention

### Technical Problem

The quantitative methods using mass spectrometry are not yet satisfying in terms of quantitative sensitivity, and in the existing circumstances, the establishment of a quantitative method using a trace amount of sample is demanded. In some cases, after a sample is prepared for mass spectrometry, when the sample is preserved for several days before measurement, measured values can be changed. The present invention has been made in view of the above, and an object thereof is to provide a method in which glucagon-secretin family peptides can be stably analyzed under conditions that a measurement sample is less prone to undergo e.g. chemical changes during preservation and practical conditions.

### Solution to Problem

The present inventor realized that in some cases, measured values of mass spectrometry were stable and in other cases, the values were not stable depending on peptide fragments to be measured. The present inventor found that when glucagon-secretin family peptides were analyzed, the selection of a peptide fragment to be analyzed was important. That is, the present analysis method is mainly characterized by cleaving the peptide bond of aspartic acid in a glucagon-secretin family peptide and selecting a peptide fragment on the N-terminal glucagon-secretin family peptide as a measuring target.

The major constitutions of the present invention are as follows.
(1) An analysis method for a glucagon-secretin family peptide in a sample, the method comprising a cleavage step of cleaving a peptide bond of aspartic acid of a glucagon-secretin family peptide in a sample to obtain peptide fragments; a separation/purification step of separating and purifying the peptide fragments cleaved in the cleavage step by liquid chromatography to elect a peptide fragment, a substance to be measured, on an N-terminal glucagon-secretin family peptide; and an analysis step of carrying out mass spectrometry to the separated and purified peptide fragments to detect the peptide fragment on the N-terminal glucagon-secretin family peptide.
(2) The analysis method according to (1), wherein the peptide bond of aspartic acid is cleaved using a cleaving agent selected from a site specific protease or acids in the cleavage step.
(3) The analysis method according to (2), wherein the cleaving agent is selected from the group consisting of endopeptidase ASP-N, formic acid, acetic acid, trifluoroacetic acid, propionic acid and a combination thereof.
(4) The analysis method according to any of (1) to (3), wherein the liquid chromatography in the separation/purification step is liquid chromatography having a flow rate of 50 nL/min to 50 µL/min.
(5) The analysis method according to any of (1) to (4), wherein the glucagon-secretin family peptide is selected from the group consisting of glucose-dependent insulinotropic polypeptide, glucagon-like peptide-1, glucagon-like peptide-2, glucagon and analogs thereof.
(6) The analysis method according to any of (1) to (5), wherein the peptide fragment to be measured is elected by selecting precursor ion mass in the separation/purification step.
(7) The analysis method according to any of (1) to (6), further comprising a step of solid-phase extraction of the peptide fragment in the separatioupurification step.
(8) The analysis method according to any of (1) to (7), wherein the separation/purification step and the analysis step are carried out by high performance liquid chromatography/mass spectrometry/mass spectrometry/mass spectrometry.
(9) A quantitative method for a glucagon-secretin family peptide in a sample, wherein a calibration curve is made using an analysis method according to any of (1) to (8) and a glucagon-secretin family peptide in a sample is quantitated using the calibration curve.
(10) The quantitative method according to (9), wherein two or more glucagon-secretin family peptides in a sample are distinguished and simultaneously quantitated by simultaneously measuring each peptide fragment of two or more glucagon-secretin family peptides.
(11) The quantitative method according to (9) or (10), wherein active and non-active glucagon-secretin family peptides in a sample are distinguished and simultaneously quantitated by simultaneously measuring each peptide fragment of an active glucagon-secretin family peptide and a non-active glucagon-secretin family peptide.
(12) The quantitative method according to any of (9) to (11), wherein a stable isotope-labeled internal standard is added to a sample to make the calibration curve.
(13) The quantitative method according to (12), wherein quantitative determination is carried out using each peak area ratio of the peptide fragment and the internal standard peptide fragment.
(14) The quantitative method according to (12) or (13), wherein the internal standard is a peptide in which one or more amino acids selected from positions 1-15 of a glucagon-secretin family peptide are substituted with stable isotope-labeled amino acid.
(15) A quantitative kit for a glucagon-secretin family peptide, the kit comprising (a) a matrix for control, (b) an internal standard or a solution thereof, (c) glucagon-secretin family peptides or standard thereof, or solutions thereof, (d) a cleaving agent, and (e) a solid phase extraction plate.
(16) The quantitative method according to (15), wherein the cleaving agent is selected from the group consisting of endopeptidase ASP-N, formic acid, acetic acid, trifluoroacetic acid, propionic acid and a combination thereof.
(17) The quantitative method according to (15) or (16), wherein the glucagon-secretin family peptide is selected from the group consisting of glucose-dependent insulinotropic polypeptide, glucagon-like peptide-1, glucagon-like peptide-2, glucagon and analogs thereof.

### Advantageous Effects of Invention

According to the present invention, glucagon-secretin family peptides can be accurately analyzed even after time elapses from preparation. Alternatively, a trace amount of glucagon-secretin family peptide in a sample can be quantitated with high sensitivity, and reproducibility of quantitative values is good.

### Brief Description of Drawings

Figure 1 is a chromatogram when LC/MS analysis is carried out immediately after two types of peptide fragments are mixed in equal amounts.
Figure 2 is a chromatogram when LC/MS analysis is carried out at 17 days after two types of peptide fragments are mixed in equal amounts and preserved in a refrigerator.
Figure 3 is a chromatogram when a GIP analog is cleaved by endopeptidase Asp-N.
Figure 4 is chromatograms when GIP is cleaved by 2% formic acid.
Figure 5 is chromatograms when GIP is cleaved by 2% acetic acid.
Figure 6 is chromatograms when GIP is cleaved by 1% trifluoroacetic acid.
Figure 7 is a chromatogram when GLP-1 is cleaved by endopeptidase Asp-N.
Figure 8 is a chromatogram when glucagon is cleaved by endopeptidase Asp-N.
Figure 9 is a chromatogram when a GLP-1 analog is cleaved by endopeptidase Asp-N.
Figure 10 is chromatograms of an active GIP peptide fragment and a corresponding internal standard when active GIP is quantitated.
Figure 11 is a graph showing a calibration curve of active GIP in the quantitative determination of active GIP.
Figure 12 is chromatograms of an active GIP peptide fragment and a corresponding internal standard when active GIP and non-active GIP are simultaneously quantitated.
Figure 13 is chromatograms of an inactive GIP peptide fragment and a corresponding internal standard when active GIP and inactive GIP are simultaneously quantitated.
Figure 14 is a graph showing a calibration curve of active GIP in the simultaneous quantitative determination of active GIP and inactive GIP.
Figure 15 is a graph showing a calibration curve of non-active GIP in the simultaneous quantitative determination of active GIP and inactive GIP.
Figure 16 is chromatograms of active GIP when active GIP and inactive GIP in a plasma sample from a diabetic are simultaneously quantitated.
Figure 17 is chromatograms of inactive GIP when active GIP and inactive GIP in a plasma sample from a diabetic are simultaneously quantitated.

### Description of Embodiments

Hereinafter the embodiments of the present invention will be described in more detail. It should be noted, however, that the present invention is not restricted to the following embodiments.

Glucagon-secretin family peptides analyzed in the present invention are peptides which have a high amino acid sequence homology to glucagon and the like. Glucagon-secretin family peptides defined herein include analogs thereof. Glucagon-secretin family peptides include glucose-dependent insulinotropic polypeptide (GIP), glucagon-like peptide-1 (GLP-1), glucagon-like peptide-2 (GLP-2), glucagon and the like. Among these, GIP and GLP-1 are known as incretin. Those which are inherent in living bodies, those which are artificially synthesized and variants are contained therein.

The glucagon-secretin family peptide analogs are peptides in which one or more amino acids are deleted, substituted or added (e.g. inserted) in amino acid sequences of glucagon-secretin family peptides, wherein the analogs have the substantially same activity as glucagon-secretin family peptides. The amino acid sequences of glucagon-secretin family peptide analogs preferably have a homology of not less than 80% to the amino acid sequences of glucagon-secretin family peptides. Such peptide design is made for the purpose of effect increases, selectivity enhancement or stability to peptide degradation, and varies depending on types of glucagon-secretin family peptides, and can be made by methods known to those of skill in the art. In glucagon-secretin family peptides, sugar chains, fatty acids, lipid, nucleic acids and the like can be bound to their peptide chains. That is, the glucagon-secretin family peptide analogs include glucagon-secretin family peptide derivatives, as well as modified, chimera and hybrid forms.

Glucose-dependent insulinotropic polypeptide (hereinafter referred to as GIP) has active GIP which consists of 42 amino acids, and is commonly expressed by GIP₁₋₄₂ [SEQ ID NO: 13]. Two amino acid residues on the N-terminal active GIP are cleaved by DPP-IV to form inactive GIP₃₋₄₂ [SEQ ID NO.: 14]. Accordingly, by measuring peptide fragments on the N-terminal GIP, active GIP and inactive GIP can be distinguished and simultaneously analyzed or quantitated. In GIP, one or more amino acids are substituted depending on species such as human, mouse or rat. Human active GIP is a peptide consisting of the following sequence, and His at position 18 is substituted with Arg in mouse and rat. Tyr-Ala-Glu-Gly-Thr-Phe-Ile-Ser-Asp-Tyr-Ser-Ile-Ala-Met-Asp-Lys-Ile-His-Gln-Gln-Asp-P he-Val-Asn-Trp-Leu-Leu-Ala-Gln-Lys-Gly-Lys-Lys-Asn-Asp-Trp-Lys-His-Asn-Ile-Thr-Gln [SEQ ID NO.: 13]

Specifically, the following are well known as GIP analogs. inactive GIP analogs include peptides in which two amino acid residues on the N-terminal GIP analogs are cleaved (the sequence ID number of a partly modified form in which the amino acid sequence is not changed is omitted).

[N-Acetyl]-GIP [N-Acetyl variant of SEQ ID NO.: 13], [N-Pyroglutamyl]-GIP [N-Pyroglutamyl variant of SEQ ID NO.: 13], [N-Glucitol]-GIP [N-Glucitol variant of SEQ ID NO.: 13], [N-Palmitate]-GIP [N-Palmitate variant of SEQ ID NO.: 13], [N-Fmoc]-GIP [N-Fmoc variant of SEQ ID NO.: 13], [N-alkyl]-GIP [N-alkyl variant of SEQ ID NO.: 13], [N-glycosyl]-GIP [N-glycosyl variant of SEQ ID NO.: 13], [N-isopropyl]-GIP [N-isopropyl variant of SEQ ID NO.: 13], [Gly2]-GIP [SEQ ID NO.: 15], [D-Ala2]-GIP [SEQ ID NO.: 16] (note that D-Ala means D-alanine, this also applies to the following)., [Aib2]-GIP [SEQ ID NO.: 17] (note that Aib means Aminoisobutylic acid, this also applies to the following.), [Phosphoserine2]-GIP [SEQ ID NO.: 18], [Sar2]-GIP [SEQ ID NO.: 19] (note that Sar means N-Methylglycine (MeGly), sarcosine, this also applies to the following.), [Pro3]-GIP [SEQ ID NO.: 20], [Hyp3]-GIP [SEQ ID NO.: 21] (note that Hyp means Hydroxyproline, this also applies to the following.), [Lys3]-GIP [SEQ ID NO.: 22], [Tyr3]-GIP [SEQ ID NO.: 23], [Phe3]-GIP [SEQ ID NO.: 24], [Ser2]-GIP [SEQ ID NO.: 113]

As glucagon-like peptide-1 (hereinafter referred to as GLP-1), GLP-1 (7-36) [SEQ ID NO.: 25] amide or GLP-1 (7-37) [SEQ ID NO.: 26] amide is commonly known as an active form thereof. International patent application No. 91/11457 has reported that GLP-1 (7-34) [SEQ ID NO.: 27] and GLP-1 (7-35) [SEQ ID NO.: 28] also have activity. Since the N-terminus of the GLP-1 precursor is generally expressed as position 1, the N-terminus of the active GLP-1 is at position 7. As is the case with GIP, active GLP-1 loses its activity by cleavage of two amino acid residues on the N-terminal side by DPP-IV to form GLP-1 (9-36) [SEQ ID NO.: 29], GLP-1 (9-37) [SEQ ID NO.: 30] and the like. Accordingly, by measuring fragments on the N-terminal GLP-1, active GLP-1 and inactive GLP-1 can be distinguished and simultaneously analyzed or quantitated. GLP-1 (7-37) is a peptide consisting of the following sequence.
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly [SEQ ID NO.: 26]

Specifically, the following are well known as GLP-1 analogs. Inactive GLP-1 analogs include peptides in which two amino acid residues on the N-terminal GLP-1 analogs are cleaved.
[Ser8]-GLP-1 (7-37) [SEQ ID NO.: 31], [Gly8]-GLP-1 (7-37) [SEQ ID NO.: 32], [Val8]-GLP-1 (7-37) [SEQ ID NO.: 33], [Glu22]-GLP-1 (7-37) [SEQ ID NO.: 34], [Lys22]-GLP-1 (7-37) [SEQ ID NO.: 35], [Val8, Glu22]-GLP-1 (7-37) [SEQ ID NO.: 36], [Val8, Lys22]-GLP-1 (7-37) [SEQ ID NO.: 37], [Gly8, Glu22]-GLP-1 (7-37) [SEQ ID NO.: 38], [Gly8, Lys22]-GLP-1 (7-37) [SEQ ID NO.: 39], [Val8, Glu30]-GLP-1 (7-37) [SEQ ID NO.: 40], [Gly8, Glu30]-GLP-1 (7-37) [SEQ ID NO.: 41], [Val8, His37]-GLP-1 (7-37) [SEQ ID NO.: 42], [Gly8, His37]-GLP-1 (7-37) [SEQ ID NO.: 43], [Arg34]-GLP-1 (7-37) [SEQ ID NO.: 44], [Lysl8]-GLP-1 (7-37) [SEQ ID NO.: 45], [Gly8, Glu22, Gly36]-GLP-1 (7-37) [SEQ ID NO.: 46], [Aib8, Aib22]-GLP-1 (7-37) [SEQ ID NO.: 47], [Aib8, Aib35]-GLP-1 (7-37) [SEQ ID NO.: 48], [Aib8, Aib22, Aib35]-GLP-1 (7-37) [SEQ ID NO.: 49], [Glu22, Glu23]-GLP-1 (7-37) [SEQ ID NO.: 50], [Gly8, Glu22, Glu23]-GLP-1 (7-37) [SEQ ID NO.: 51], [Val8, Glu22, Glu23]-GLP-1 (7-37) [SEQ ID NO.: 52], [Val8, Glu22, Val25]-GLP-1 (7-37) [SEQ ID NO.: 53], [Val8, Glu22, Ile33]-GLP-1 (7-37) [SEQ ID NO.: 54], [Val8, Glu22, Val25, Ile33]-GLP-1 (7-37) [SEQ ID NO.: 55], and the GLP-1 (7-36) type in which the residue at position 37 thereof is deleted, and the GLP-1 (7-35) type in which the residues at positions 36 and 37 thereof are deleted

Further, exendin is known as a GLP-1 receptor agonist, and is also included in GLP-1 analogs. Exendin analogs are known, such as Exendin-3 [SEQ ID NO.: 56], Exendin-3 amide [the C-terminal amide derivative of the same sequence as SEQ ID NO.: 56], Exendin-4 [SEQ ID NO.: 57], Exendin-4 amide [the C-terminal amide derivative of the same sequence as SEQ ID NO.: 57], Exendin-4 acid [an acid variant of the same sequence as SEQ ID NO.: 57], Exendin-4-LysLysLysLysLys [SEQ ID NO.: 58], Exendin-4-LysLysLysLysLysLys [SEQ ID NO.: 59], Exendin-4 (1-30) [SEQ ID NO.: 60], Exendin-4 amide (1-30) [the C-terminal amide derivative of the same sequence as SEQ ID NO.: 60], Exendin-4 (1-28) [SEQ ID NO.: 61], Exendin-4 amide (1-28) [the C-terminal amide derivative of the same sequence as SEQ ID NO.: 61], ¹⁴Leu²⁵Phe-Exendin-4 amide [the C-terminal amide derivative of the same sequence as SEQ ID NO.: 62] and ¹⁴Leu²⁵Phe-Exendin-4 amide (1-28) [the C-terminal amide derivative of SEQ ID NO.: 63]. Other analogs are specifically disclosed in e.g. International Publication No. WO 2009/035540. Exendin-4 is a biologically active peptide having GLP-1-like activity, which is detected from salivary gland secretion of Heloderma suspectum.

Glucagon-like peptide-2 (hereinafter referred to as GLP-2) is an intestinotrophic peptide hormone with 33 amino acids which is formed via post-translational processing of proglucagon, and is commonly expressed by GLP-2 [SEQ ID NO.: 64]. As is the case with GIP, GLP-2 loses its activity by cleavage of two amino acid residues on the N-terminal side by DPP-IV to form inactive GLP-2 (3-33) [SEQ ID NO.: 65]. By measuring fragments on the N-terminal GLP-2, active GLP-2 and inactive GLP-2 can be distinguished and simultaneously analyzed or quantitated. Specifically, the following are well known as GLP-2 analogs. Inactive GLP-2 analogs include peptides in which two amino acid residues on the N-terminal GLP-2 peptides are hydrolyzed.
[Ser2]-GLP-2 [SEQ ID NO.: 66], [Gly2]-GLP-2 [SEQ ID NO.: 67], [Val2]-GLP-2 [SEQ ID NO.: 68]

Glucagon is a peptide hormone consisting of 29 amino acids, and is commonly expressed by glucagon [SEQ ID NO.: 69]. Specifically, the following are well known as glucagon analogs.
[Arg12]-Glucagon [SEQ ID NO.: 70], [Arg12, Lys20]-Glucagon [SEQ ID NO.: 71], [Arg12, Lys24]-Glucagon [SEQ ID NO.: 72], [Arg12, Lys29]-Glucagon [SEQ ID NO.: 73], [Glu9]-Glucagon [SEQ ID NO.: 74], [Glu9, Glul6, Lys29]-Glucagon [SEQ ID NO.: 75], [Lys13, Glu17]-Glucagon [SEQ ID NO.: 76], [Glu20, Lys24]-Glucagon [SEQ ID NO.: 77]

Samples include biological samples such as blood, serum, plasma, urine, saliva, exudates and tissue extracts, and in vitro samples such as medicines and cell culture fluid. Glucagon-secretin family peptides can be quantitated using a sample with preferably 5 µL to 5 mL and a concentration range of 0.1 pM to 500 pM, and can be quantitated using a sample with more preferably 10 µL to 3 mL and a concentration range of 0.5 pM to 200 pM. When glucagon-secretin family peptides can be quantitated using a sample with 5 mL or less, burden to patients can be reduced because the amount of blood to be collected can be lowered, and extraction operation is not complicated. When a sample is plasma, the above-mentioned quantitative determination can be carried out using plasma with preferably 5 µL to 500 µL and a concentration range of 0.1 pM to 500 pM, and can be carried out using plasma with more preferably 10 µL to 300 µL and a concentration range of 0.5 pM to 200 pM.

Peptide fragments of a glucagon-secretin family peptide mean peptides after the glucagon-secretin family peptide is cleaved by some methods, and indicate consecutive sections of the amino acid sequence of the glucagon-secretin family peptide. In the present analysis method, the peptide bond of aspartic acid (Asp) in the amino acid sequence of a glucagon-secretin family peptide is cleaved, and among these peptide fragments, the peptide fragment on the N-terminal glucagon-secretin family peptide is subjected to mass spectrometry. About this cleavage, the N-terminal side of aspartic acid can be cleaved or the C-terminal side thereof can be cleaved. By using such peptide fragment, stable measured values are easily obtained, and sensitivity is improved. In addition, the analysis using mass spectrometry can be carried out without using antibodies. Further, multiply-charged ions are less prone to occur, and influences due to types of species can be reduced. In GIP for example, positions 1-17 of the amino acid sequence have a high homology and are identical in human, rat and mouse. For preservation stability, it is important that a peptide fragment to be measured not comprise an oxidizable amino acid. There are methionine (Met), tryptophan (Trp) and cysteine (Cys) as oxidizable amino acids. Peptide fragments on the N-terminal glucagon-secretin family peptides do not comprise the above-mentioned amino acids, and preservation stability is good. When glucagon-secretin family peptides are different, peptide fragments in the present method are different. It is thought that a peptide having the same sequence as a peptide on the N-terminal side cleaved at aspartic acid of a glucagon-secretin family peptide does not exist in a living body. Because of the above two points, peptide fragments on the N-terminal glucagon-secretin family peptides are distinguished by mass spectrometry and can be analyzed with high specificity.

The peptide bond of aspartic acid in amino acid sequences can be cleaved using substances (cleaving agents) which cleave a peptide bond. The substances which cleave a peptide bond can include site specific proteases, acids or the like. An example of site specific proteases is endopeptidase Asp-N. Endopeptidase Asp-N hydrolyzes the peptide bond on the N-terminal side of aspartic acid (Asp). As the acids, there are organic acids, inorganic acids and the like. Examples of organic acids include carboxylic acids such as formic acid, acetic acid, propionic acid, hexanoic acid, citric acid, chloroacetic acid, dichloroacetic acid, trichloroacetic acid, trifluoroacetic acid (TFA), benzoic acid, salicylic acid, oxalic acid, succinic acid, malonic acid, phthalic acid, tartaric acid, malic acid and glycolic acid; sulfonic acids such as methanesulfonic acid, benzenesulfonic acid, p-toluenesulfonic acid and trifluoromethanesulfonic acid; and the like. Examples of inorganic acids include hydrochloric acid, hydrobromic acid, sulfuric acid, phosphoric acid, tetrafluoroboric acid, perchloric acid, periodic acid and the like. These acids can be used individually or two or more acids can be properly combined. Among these cleaving agents, any one selected from the group consisting of endopeptidase ASP-N, formic acid, acetic acid, trifluoroacetic acid, propionic acid and a combination thereof is preferred. When an acid is used as a cleaving agent, a 1 to 2% organic acid is commonly used in an amount of preferably 10 µL to 3 mL, and further preferably 100 µL to 1 mL per 5 nmol of glucagon-secretin family peptide. The acidity for cleavage is preferably pH 1 to 5 and further preferably pH 2 to 4. By using these acids, the peptide bond on the C-terminal side or the N-terminal side of aspartic acid (Asp) can be hydrolyzed, and analysis and quantitative determination can be carried out using the hydrolyzed peptide fragment on the N-terminal glucagon-secretin family peptide.

When a glucagon-secretin family peptide is cleaved by a site specific protease, reaction temperature is commonly 25 to 45°C, and preferably 35 to 40°C. Reaction time is commonly for 4 to 24 hours, and preferably for 10 to 18 hours. When a glucagon-secretin family peptide is cleaved by an acid, reaction temperature is commonly 60 to 120°C, and preferably 95 to 110°C. Reaction time is commonly for 30 minutes to 24 hours, and preferably for 2 to 20 hours. A solvent can be used for any reaction, and any solvent which does not inhibit reactions can be used.

When the peptide bonds of aspartic acid (Asp) in two or more glucagon-secretin family peptides are cleaved, by measuring peptide fragments on the N-terminal side, two or more glucagon-secretin family peptides can be distinguished and simultaneously analyzed. In this case, it is preferred to select two or more glucagon-secretin family peptides which have the same extraction conditions.

The amino acid sequences on the N-terminal side are important for physiological activity of glucagon-secretin family peptides. That is, in some glucagon-secretin family peptides, two amino acid residues on the N-terminal side are lost by an enzyme such as dipeptidil peptidase-4 (hereinafter, referred to as DPP-4) to lose activity. In GIP₁₋₄₂, active GIP, for example, two residues (Tyr at position 1 and Ala at position 2) on the N-terminal side are cleaved by DPP-4 to form GIP₃₋₄₂ and its activity is lost. Accordingly, by measuring peptide fragments on the N-terminal glucagon-secretin family peptides, active glucagon-secretin family peptides in which two residues on the N-terminal side remain and inactive glucagon-secretin family peptides in which two residues on the N-terminal side are lost can be distinguished and quantitated. When the active type and the inactive type can be distinguished and quantitated, both active and inactive glucagon-secretin family peptides can be simultaneously quantitated.

To improve the precision of measurement by mass spectrometry, the number of amino acids in a peptide to be measured is preferably 5 to 14, and more preferably 6 to 9. The peptide fragments in the present method are in the value range, and are suitable for measurement by mass spectrometry.

Peptide fragments on the N-terminal GIP or analogs thereof can be expressed by the following expression.
Expression: X₁-X₂-X₃-Gly-Thr-Phe-Ile-Ser-X₄ [SEQ ID NO.: 78]
[wherein, X₁ means Tyr, D-Tyr, N-Acetyl-Tyr, N-Pyroglutamyl-Tyr, N-Glucitol-Tyr, N-Palmitate-Tyr, N-Fmoc-Tyr, N-alkyl-Tyr, N-glycosyl-Tyr, N-isopropyl-Tyr or deletion,
X₂ means Ala, D-Ala, Gly, Ser, 2-Aminobutylic acid, Aminoisobutylic acid, Phosphoserine, Sarcosine, or deletion,
X₃ means Glu, D-Glu, Pro, Hydroxyproline, Lys, Tyr, or Phe, and
X₄ means Asp or deletion.]

Specific examples of peptide fragments on the N-terminal GIP or analogs thereof are as follows. Those in which two amino acid residues on the N-terminal side of the following peptide fragments on the N-terminal GIP or analogs thereof are deleted are inactive peptide fragments.
Tyr-Ala-Glu-Gly-Thr-Phe-Ile-Ser [SEQ ID NO.: 1], N-Acetyl-Tyr-Ala-Glu-Gly-Thr-Phe-Ile-Ser [N-Acetyl variant of SEQ ID NO.: 1], N-Pyroglutamyl-Tyr-Ala-Glu-Gly-Thr-Phe-Ile-Ser [N-Pyroglutamyl variant of SEQ ID NO.: 1], N-Glucitol-Tyr-Ala-Glu-Gly-Thr-Phe-Ile-Ser [N-Glucitol variant of SEQ ID NO.: 1], N-Palmitate-Tyr-Ala-Glu-Gly-Thr-Phe-Ile-Ser [N-Palmitate variant of SEQ ID NO.: 1], N-Frnoc-Tyr-Ala-Glu-Gly-Thr-Phe-Ile-Ser [N-Fmoc variant of SEQ ID NO.: 1], N-alkyl-Tyr-Ala-Glu-Gly-Thr-Phe-Ile-Ser [N-alkyl variant of SEQ ID NO.: 1], N-glycosyl-Tyr-Ala-Glu-Gly-Thr-Phe-Ile-Ser [N-glycosyl variant of SEQ ID NO.: 1], N-isopropyl-Tyr-Ala-Glu-Gly-Thr-Phe-Ile-Ser [N-isopropyl variant of SEQ ID NO.: 1], Tyr-Gly-Glu-Gly-Thr-Phe-Ile-Ser [SEQ ID NO.: 79], Tyr-Ser-Glu-Gly-Thr-Phe-Ile-Ser [SEQ ID NO.: 5], Tyr-D-Ala-Glu-Gly-Thr-Phe-Ile-Ser [SEQ ID NO.: 80], Tyr-Abu-Glu-Gly-Thr-Phe-Ile-Ser [SEQ ID NO.: 81] (note that Abu means 2-Aminobutylic acid, this also applies to the following.), Tyr-Aib-Glu-Gly-Thr-Phe-Ile-Ser [SEQ ID NO.: 82], Tyr-Phosphoserine-Glu-Gly-Thr-Phe-Ile-Ser [SEQ ID NO.: 83], Tyr-Sar-Glu-Gly-Thr-Phe-Ile-Ser [SEQ ID NO.: 84], Tyr-Ala-Pro-Gly-Thr-Phe-Ile-Ser [SEQ ID NO.: 85], Tyr-Ala-Hyp-Gly-Thr-Phe-Ile-Ser [SEQ ID NO.: 86], Tyr-Ala-Lys-Gly-Thr-Phe-Ile-Ser [SEQ ID NO.: 87], Tyr-Ala-Tyr-Gly-Thr-Phe-Ile-Ser [SEQ ID NO.: 88], Tyr-Ala-Phe-Gly-Thr-Phe-Ile-Ser [SEQ ID NO.: 89], Tyr-Ala-Glu-Gly-Thr-Phe-Ile-Ser-Asp [SEQ ID NO.: 2], N-Acetyl-Tyr-Ala-Glu-Gly-Thr-Phe-lle-Ser-Asp [N-Acetyl variant of SEQ ID NO.: 2], N-Pyroglutamyl-Tyr-Ala-Glu-Gly-Thr-Phe-Ile-Ser-Asp [N-Pyroglutamyl variant of SEQ ID NO.: 2], N-Glucitol-Tyr-Ala-Glu-Gly-Thr-Phe-Ile-Ser-Asp [N-Glucitol variant of SEQ ID NO.: 2], N-Palmitate-Tyr-Ala-Glu-Gly-Thr-Phe-Ile-Ser-Asp [N-Palmitate variant of SEQ ID NO.: 2], N-Fmoc-Tyr-Ala-Glu-Gly-Thr-Phe-Ile-Ser-Asp [N-Fmoc variant of SEQ ID NO.: 2], N-alkyl-Tyr-Ala-Glu-Gly-Thr-Phe-Ile-Ser-Asp [N-alkyl variant of SEQ ID NO.: 2], N-glycosyl-Tyr-Ala-Glu-Gly-Thr-Phe-Ile-Ser-Asp [N-glycosyl variant of SEQ ID NO.: 2], N-isopropyl-Tyr-Ala-Glu-Gly-Thr-Phe-Ile-Ser-Asp [N-isopropyl variant of SEQ ID NO.: 2], Glu-Gly-Thr-Phe-Ile-Ser-Asp [SEQ ID NO.: 4], Tyr-Gly-Glu-Gly-Thr-Phe-Ile-Ser-Asp [SEQ ID NO.: 90], Tyr-Ser-Glu-Gly-Thr-Phe-Ile-Ser-Asp [SEQ ID NO.: 91], Tyr-D-Ala-Glu-Gly-Thr-Phe-Ile-Ser-Asp [SEQ ID NO.: 92], Tyr-Abu-Glu-Gly-Thr-Phe-Ile-Ser-Asp [SEQ ID NO.: 93], Tyr-Aib-Glu-Gly-Thr-Phe-Ile-Ser-Asp [SEQ ID NO.: 94], Tyr-Phosphoserine-Glu-Gly-Thr-Phe-Ile-Ser-Asp [SEQ ID NO.: 95], Tyr-Sar-Glu-Gly-Thr-Phe-Ile-Ser-Asp [SEQ ID NO.: 96], Tyr-Ala-Pro-Gly-Thr-Phe-Ile-Ser-Asp [SEQ ID NO.: 97], Tyr-Ala-Hyp-Gly-Thr-Phe-Ile-Ser-Asp [SEQ ID NO.: 98], Tyr-Ala-Lys-Gly-Thr-Phe-Ile-Ser-Asp [SEQ ID NO.: 99], Tyr-Ala-Tyr-Gly-Thr-Phe-Ile-Ser-Asp [SEQ ID NO.: 100], Tyr-Ala-Phe-Gly-Thr-Phe-Ile-Ser-Asp [SEQ ID NO.: 101]

Peptide fragments on the N-terminal GLP-1 or analogs thereof can be expressed by the following expression.
Expression: His-X₈-X₉-Gly-Thr-Phe-Thr-Ser-X₁₅ [SEQ ID NO.: 102]
[wherein, X₈ means Ala, Gly, Ser, Thr, Leu, Ile, Val, Glu, Lys, or Aib,
X₉ means Glu, Gly, or Lys, and
X₁₅ means Asp or deletion.]

Specific examples of peptide fragments on the N-terminal GLP-1 or analogs thereof are as follows. Those in which two amino acid residues on the N-terminal side of the following peptide fragments on the N-terminal GLP-1 or analogs thereof are deleted are inactive peptide fragments.
His-Ala-Glu-Gly-Thr-Phe-Thr-Ser [SEQ ID NO.: 6], His-Ser-Glu-Gly-Thr-Phe-Thr-Ser [SEQ ID NO.: 10], His-Gly-Glu-Gly-Thr-Phe-Thr-Ser [SEQ ID NO.: 103], His-Val-Glu-Gly-Thr-Phe-Thr-Ser [SEQ ID NO.: 104], His-Aib-Glu-Gly-Thr-Phe-Thr-Ser [SEQ ID NO.: 105], His-Ala-Glu-Gly-Thr-Phe-Thr-Ser-Asp [SEQ ID NO.: 7], His-Ser-Glu-Gly-Thr-Phe-Thr-Ser-Asp [SEQ ID NO.: 106], His-Gly-Glu-Gly-Thr-Phe-Thr-Ser-Asp [SEQ ID NO.: 107], His-Val-Glu-Gly-Thr-Phe-Thr-Ser-Asp [SEQ ID NO.: 108], His-Aib-Glu-Gly-Thr-Phe-Thr-Ser-Asp [SEQ ID NO.: 109]

Specific examples of peptide fragments on the N-terminal GLP-2 are as follows. Those in which two amino acid residues on the N-terminal side of the following peptide fragments on the N-terminal GLP-2 are deleted are inactive peptide fragments. His-Ala-Asp-Gly-Ser-Phe-Ser [SEQ ID NO.: 110], His-Ala-Asp-Gly-Ser-Phe-Ser-Asp [SEQ ID NO.: 111]

Specific examples of peptide fragments on the N-terminal glucagon are as follows. His-Ser-Gln-Gly-Thr-Phe-Thr-Ser [SEQ ID NO.: 11], His-Ser-Gln-Gly-Thr-Phe-Thr-Ser-Asp

### [SEQ ID NO.: 12]

It is preferred that before the analysis step by a mass spectrometer, peptide fragments of glucagon-secretin family peptides in a sample be subjected to treatment to remove interfering substances such as protein, peptides and biological low molecular weight substances which exist in large numbers in the sample. Quantitative determination sensitivity or precision in the analysis step can be further increased by this treatment. Treatment to remove these interfering substances can be properly carried out by using one or two or more means, such as concentration, dissolution in different solvents, extraction, crystallization, centrifugation and purification, in combination by those of skill in the art. Treatment to remove interfering substances can be carried out either before or after the cleavage step or both.

Examples of extraction methods as treatment to remove the above interfering substances can include solid-phase extraction and the like. The solid-phase extraction can be carried out by publicly known methods, for example, glucagon-secretin family peptides are retained on a solid phase, and the glucagon-secretin family peptides retained on the solid phase are then eluted by an elution solvent. In addition, one or two or more of solid phases can be used in combination. As a preferred combination of solid phases, solid phases with different properties are used, for example, a combination of an ion exchange type of solid phase extraction tool and a reversed phase type of solid phase extraction tool. By doing this, a wide variety of interfering substances can be effectively removed. An anion exchange type of solid phase extraction tool or a cation exchange type of solid phase extraction tool can be used, and can be properly selected depending on types of glucagon-secretin family peptides by those of skill in the art. In a solid phase extraction method, a commercially available cartridge or mini column or 96-well solid phase extraction plate or the like in which the above-mentioned solid phase is filled can be used. Concentration operation can be carried out by e.g. vacuum concentration etc.

Purification carried out as treatment to remove the above interfering substances can be carried out by liquid chromatography and the like. In liquid chromatography, two phases, a mobile phase and a stationary phase, are involved. In reversed phase chromatography, for example, a water-acetonitrile mixed liquid as a mobile phase and a stationary phase in which the octadecylsilyl group (C18) is bound to a silica gel support are used, and separation from other components is carried out due to a difference in polarity between the two and an interaction of polarity of a peptide to be measured. Interfering substances and a peptide to be measured can be separated by reversed phase chromatography or ion exchange chromatography, or a combination thereof utilizing the water-solubility and isoelectric point of the peptide. Especially when a column switching method is used, interfering substances can be efficiently removed using several types of solid phases. Other purification methods can include size exclusion chromatography, electrophoresis and the like. In the present method, purification by liquid chromatography is essential, and one or two or more of other purification can be combined. As liquid chromatography, HPLC, UPLC, UHPLC, nanoflow-LC and the like are known. When a sample comprising peptide fragments obtained in the above-mentioned cleavage step is separated and purified by liquid chromatography, a column is heated (preferably 40 to 60°C), and methanol comprising 0.1% formic acid can be used as a mobile phase. However, the above-mentioned conditions vary depending on samples to be treated, and are not restricted. The flow rate of liquid chromatography is preferably 50 nL/min to 50 µL/min, and further preferably 100 nL/min to 1 µL/min. When the flow rate is low, very fine charged droplets can be produced by electrospray ionization, and desolvation can be carried out with high efficiency. The inner diameter of a column used for liquid chromatography is preferably 50 µm or more and less than 1 mm, and further preferably 50 µm or more and 800 µm or less. When a column with a small inner diameter is used, diffusion of peptide fragments to be measured in the column can be prevented, and an improvement in measurement sensitivity can be intended.

A peptide fragment separated and eluted by liquid chromatography is measured by a mass spectrometer. In mass spectrometry, a substance to be measured is firstly ionized by any means, and the mass/charge ratio (m/z) of the ion and the ion amount of the mass are measured. Accordingly, there exist various combinations of ionization and ion measurement in mass spectrometers. For example, mass spectrometers having various functions are known, such as mass spectrometers by the electrospray ionization (ESI) method or the atmospheric pressure chemical ionization (APCI) method, mass spectrometers connected to liquid chromatography (LC/MS, LC/MS/MS, LC/MS/MS/MS etc.), mass spectrometers in which two or more mass spectrometers are connected (MS/MS, MS/MS/MS etc.), tandem mass spectrometers (tandem MS), triple-quadrupole mass spectrometers in which a collision cell is provided between two transmission quadrupole mass spectrometers placed in series (LC/MS/MS etc.), mass spectrometers utilizing an ion trap function (MS/MS/MS etc.), or quadrupole time-of-flight mass spectrometers. In LC/MS/MS, a specific precursor ion to a peptide to be quantitated is selected, and e.g. argon is then collided to dissociate the ion, and a new ion group is generated. One or more ions from this new ion group (product ion) are analyzed by a mass spectrometer (MS). Therefore, quantitative determination can be carried out with high specificity. In MS/MS/MS which has been developed in recent years, the establishment of a quantitative method with higher specificity is demanded. In MS/MS/MS, measurement can be carried out by setting a primary product ion generated from a precursor ion and further a secondary product ion generated from the primary product ion, and thus, it is advantageous for measurement in a system in which similar amino acid sequences coexist. The precursor ion is an ion generated by the above-mentioned electrospray ionization, atmospheric pressure chemical ionization and the like, and is a precursor ion of a product ion.

In an embodiment of the present invention, the separation step and the analysis step can be carried out by a method in which liquid chromatography and mass spectrometry are combined. Specific examples of such methods include liquid chromatography/tandem mass spectrometry (LC/MS/MS, LS/MS/MS/MS etc.), a liquid chromatography/mass spectrometry (LC/MS) method and the like. It is preferred that nanoFlow LC-MS/MS/MS be used, in which a triple-quadrupole mass spectrometer with an ion trap function is connected to liquid chromatography, but it is not restricted in the present invention.

A mass spectrum is obtained by mass spectroscopy of peptide fragments, and is a spectrum in which the m/z is taken along the abscissa and the detected intensity is taken along the ordinate. The m/z is a value obtained by dividing the ion mass by the unified atomic mass unit and further dividing the obtained value by the charge number of ion. By entering a specific m/z to a substance to be measured into a device, a mass spectrometer can specifically introduce only ions with the m/z into a detector, and thus, analysis can be carried out with high specificity. Examples of peptide fragments of glucagon-secretin family peptides are shown in Table 1. As is the case with other glucagon-secretin family peptides, a specific m/z of a precursor ion can be set by methods well known to those of skill in the art. When MS/MS or MS/MS/MS is used, quantitative determination can be carried out with high specificity by setting the m/z of 1 to 5 fragment ions and preferably 1 to 3 fragment ions in the same manner.

| SEQ ID NO. | Abbreviated name | Amino acid sequence | | Precursor ion m/z |
|---|---|---|---|---|
| 1 | GIP₁₋₈ | Tyr-Ala-Glu-Gly-Thr-Phe-lle-Ser | | 887.4±0.50 |
| 2 | GIP₁₋₉ | Tyr-Ala-Glu-Gly-Thr-Phe-Ile-Ser-Asp | | 1002.4±0.50 |
| 3 | GIP₃₋₈ | | Glu-Gly-Thr-Phe-Ile-Ser | 653.3±0.50 |
| 4 | GIP₃₋₉ | | Glu-Gly-Thr-Phe-Ile-Ser-Asp | 768.3±0.50 |
| 5 | 2S-GIP₁₋₈ | Tyr-Ser-Glu-Gly-Thr-Phe-Ile-Ser | | 903.4±0.50 |
| 6 | GLP-1 (7-14) | His-Ala-Glu-Gly-Thr-Phe-Thr-Ser | | 849.4±0.50 or 425.2±0.50 |
| 7 | GLP-1 (7-15) | His-Ala-Glu-Gly-Thr-Phe-Thr-Ser-Asp | | 964.4±0.50 or 482.7±0.50 |
| 8 | GLP-1 (9-14) | | Glu-Gly-Thr-Phe-Thr-Ser | 641.3±0.50 or 321.1±0.50 |
| 9 | GLP-1 (9-15) | | Glu-Gly-Thr-Phe-Thr-Ser-Asp | 756.3+0.50 or 378.7+0.50 |
| 10 | 8S-GLP-1 (7-14) | His-Ser-Glu-Gly-Thr-Phe-Thr-Ser | | 865.4+0.50 or 433.2+0.50 |
| 11 | Glucagon₁₋₈ | His-Ser-Gln-Gly-Thr-Phe-Thr-Ser | | 864.4±0.50 or 432.7±0.50 |
| 12 | Glucagon₁₋₉ | His-Ser-Gln-Gly-Thr-Phe-Thr-Ser-Asp | | 979.4±0.50 or 490.2±0.50 |

In another embodiment of the present invention, glucagon-secretin family peptides or analogs thereof in a sample are quantitated using a calibration curve. Using standard solutions diluted in stages, a peptide fragment derived from a standard is measured by a mass spectrometer, and a calibration curve can be made according to general methods to those of skill in the art. It is preferred that in a calibration curve, the accuracy be within ± 20% at the lower limit of quantification and within ± 15% at other concentrations, and the correlation coefficient be 0.990 or more. When quantitative determination is carried out using a calibration curve, (a) the absolute calibration method in which quantitative determination is carried out using a peak area detected by injecting a standard in a fixed amount, (b) the internal standard method in which quantitative determination is carried out using a peak area ratio detected between a sample to be measured and a standard (an internal standard) to be added wherein the standard is different from the sample, and the like are used. It is preferred that a calibration curve be made using the internal standard method to increase quantitative determination precision. When an internal standard is used, for example, a ratio between an area peak of a glucagon-secretin family peptide in a sample and an area peak of the internal standard is calculated, and by plotting this ratio on a graph, a calibration curve with high reliability can be made. In addition, by using standard solutions prepared by dissolving a standard, human endogenous glucagon-secretin family peptides in a sample can be measured, and quantitative determination can be carried out even in a concentration range of not more than a standard concentration. The lower limit of quantification is the weakest concentration of standard concentration in a sample among samples in which a standard used to make a calibration curve is added, and means that the above-mentioned accuracy is within ± 20%. The accuracy of a calibration curve is calculated by dividing a value obtained by subtracting an addition concentration from a quantitative value by the addition concentration. The above-mentioned correlation coefficient can be calculated by using spreadsheet software. Using a created calibration curve, glucagon-secretin family peptides in a sample can be quantitated.

As an internal standard, for example, preferred is one in which one or more amino acids of a glucagon-secretin family peptide to be measured are substituted with stable isotope-labeled amino acids. Any amino acid selected from positions 1-15 of amino acids to be measured is further preferably substituted with a stable isotope-labeled amino acid. When a stable isotope-labeled peptide is used, for example, an internal standard can be obtained by e.g. labeling Phe with a stable isotope. Phe frequently exists at any of positions 1-15 of glucagon-secretin family peptides. Phe, for example, exists at position 6 of GIP₁₋₄₂, position 12 of GLP-1 (7-36), position 6 of GLP-2 and position 6 of glucagon. Phe is an amino acid suitable for stable isotope labeling, and can cause a difference of 10 Da or more from a peptide to be originally measured, and thus, cross-talk at the time of quantitative determination by mass spectrometry can be minimized. Further, e.g. Leu, Ile, Val, Ala, Tyr, Glu, Gly, Thr, Ser or Pro is an amino acid suitable for stable isotope labeling. Stable isotopes include ²H, ¹³C, ¹⁵N, ¹⁸O and the like, and these isotopes can be used individually or two or more isotopes can be used in combination. Further, there are a method in which ¹⁸O is introduced by hydrolase cleavage in H₂¹⁸O, a method in which stable isotope labeling is carried out using e.g. a commercially available stable isotope-labeled reagent, and the like. A stable isotope-labeled peptide is chemically identical with a non-labeled peptide except that the mass of a peptide to be quantitated and the mass of labeled amino acid are different, and both peptides express identical behaviors in LC-MS/MS measurement. Therefore, influences to ionization by interfering substances can be removed, and the stable isotope-labeled peptide can be advantageously used as an internal standard peptide. In all methods, a substance to be measured and an internal standard have a mass difference, and thus are detected as different peaks by e.g. LC/MS, and quantitative determination can be carried out based on an area or height ratio of both peaks.

A stable isotope-labeled internal standard can be the whole amino acid sequence of a glucagon-secretin family peptide, or can be a peptide fragment to be measured. An internal standard, in which part of the whole amino acid sequence is labeled, is preferred to increase accuracy of quantitative values. Supposing that an internal standard in which part of a peptide fragment to be measured is labeled is used, extraction efficiency in pretreatment up to the cleavage step will not be always the same as that of a glucagon-secretin family peptide to be quantitated. In addition, digestion efficiency by site specific proteases or acids is not always the same as that of a glucagon-secretin family peptide to be quantitated. These extraction efficiency and digestion efficiency vary in each sample, and thus, it is difficult to completely correct a glucagon-secretin family peptide to be quantitated using an internal standard in which part of a peptide fragment to be measured is labeled. Accordingly, when part of the whole amino acid sequence is labeled and added to a sample, the amount of glucagon-secretin family peptide can be corrected in the entire process of pretreatment, and thus, accuracy of quantitative values can be increased. As internal standard, not only stable isotope-labeled peptides but also peptides in which one or more amino acids of a peptide fragment to be measured are altered or peptides in which the order of the amino acid sequence of a peptide fragment to be measured is changed can be used.

An embodiment of the present invention constitutes a kit for quantitating glucagon-secretin family peptides or analogs thereof in a sample. This kit comprises (a) a matrix for control, (b) an internal standard or a solution thereof, (c) glucagon-secretin family peptides or solutions thereof, (d) a cleaving agent and (e) a solid phase extraction plate. The above (d) cleaving agent is as described in paragraph 24, and any one selected from the group consisting of endopeptidase ASP-N, formic acid, acetic acid, trifluoroacetic acid, propionic acid and a combination thereof is preferred. To the kit, a buffer for treating a cleaving agent can be added as needed. The method for using this kit will be described below. Firstly, to a sample and (a) a matrix for control, (b) an internal standard or a solution thereof is added, and for a calibration curve, (c) glucagon-secretin family peptides or solutions thereof are further added in certain amounts. After that, peptide fragments are obtained using (d) a cleaving agent, and extraction operation is then carried out using (e) a solid phase extraction plate. Peptide fragments of a calibration curve and in an extraction sample obtained from the sample are measured, and glucagon-secretin family peptides in the sample can be quantitated by applying the ratio between an area peak of a glucagon-secretin family peptide obtained from the sample and an area peak of the internal standard to the calibration curve.

### Examples

The present invention will be described in more detail by way of the following examples. It should be noted, however, that the present invention is not restricted thereto, and changes can be made without departing from the scope of the present invention.

Test 1 Comparison of measuring methods for glucagon-secretin family peptides

### Method

The stability of peptide fragments to be measured of GIP, a glucagon-secretin family peptide, was compared. To the following (a) and (b) peptide fragments synthesized by solid phase synthesis, 0.1% TFA-20% acetonitrile was added to obtain a 2.0 µM solution. This solution was measured by LC/MS/MS (Applied Biosystems: QSTAR (registered trademark) Elite). The measurement was carried out immediately after preparation and at 17 days after preparation.
(a) GIP₁₋₈: GIP₁₋₈, in which aspartic acid at position 9 of GIP₁₋₄₂ was cleaved on the N-terminal side, was used. The amino acid sequence of GIP₁₋₈ is Tyr-Ala-Glu-Gly-Thr-Phe-Ile-Ser [SEQ ID NO.: 1].
(b) GIP₁₋₁₆: GIP₁₋₁₆, in which lysine at position 16 of GIP₁₋₄₂ was cleaved on the C-terminal side, was used. When GIP₁₋₄₂ is cleaved by trypsin, lysine at position 16 is cleaved on the C-terminal side. The amino acid sequence of GIP₁₋₁₆ is Tyr-Ala-Glu-Gly-Thr-Phe-Ile-Ser-Asp-Tyr-Ser-Ile-Ala-Met-Asp-Lys [SEQ ID NO.: 112].

### Results

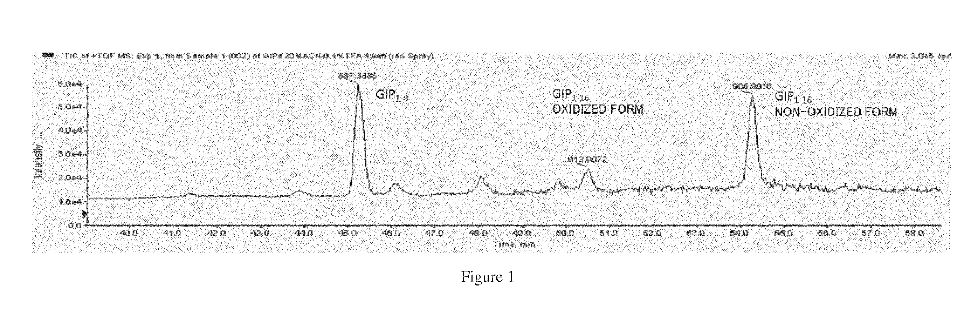

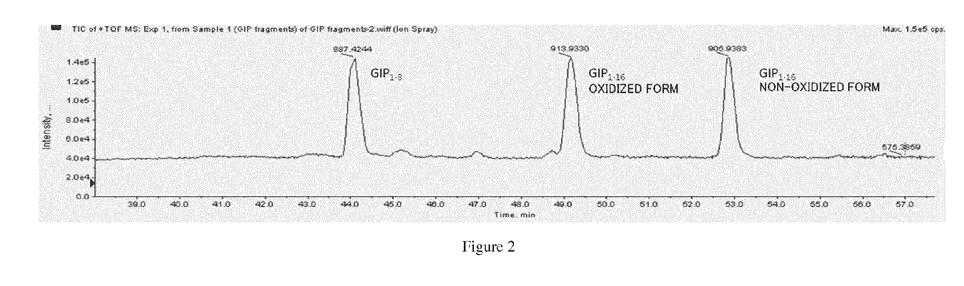

The measured values immediately after preparation are shown in Figure 1, and the measured values at 17 days after preparation are shown in Figure 2.

About GIP₁₋₈, one peak was observed (m/z 887.4 ([M + H]⁺)). About GIP₁₋₁₆, two peaks were observed (m/z 905.9 ([M + 2H]²⁺) and m/z 913.9 ([M + 2H]²⁺)). One peak observed in GIP₁₋₁₆ enlarged with time during refrigerated preservation, and the peak intensity ratio between the peak and another peak was almost 1 : 1 at 17 days after preparation. The peak changing with time was the peak of an oxidized form in which methionine (Met) of GIP₁₋₁₆ was oxidized. The peak enlarges with time, which shows that the molecular weight of the peptide changes with time and concentration in a sample cannot be accurately measured. The peptide fragment of GIP₁₋₈ was more stable than the peptide fragment of GIP₁₋₁₆.

### <Example 1> Measuring method for GIP analogs

### Method

An embodiment for measuring a GIP analog (2S-GIP-NH₂) [the C-terminal amide derivative of SEQ ID NO.: 113], a glucagon-secretin family peptide, is shown. This GIP analog is a GIP analog in which alanine at position 2 of active GIP is substituted with serine and the C-terminus thereof is amidated. As a cleaving agent, ASP-N was used. Such GIP analog was dissolved with 50 mM Tris/HCl and a 2.5 mM ZnSO₄ solution (pH 8.0). To this solution, 1 µg of Asp-N was added, and the solution was incubated at 37°C for 15 hours to cleave such GIP analog. After that, the sample was analyzed by LC/MS (LCQ Deca XP Plus).

### Results

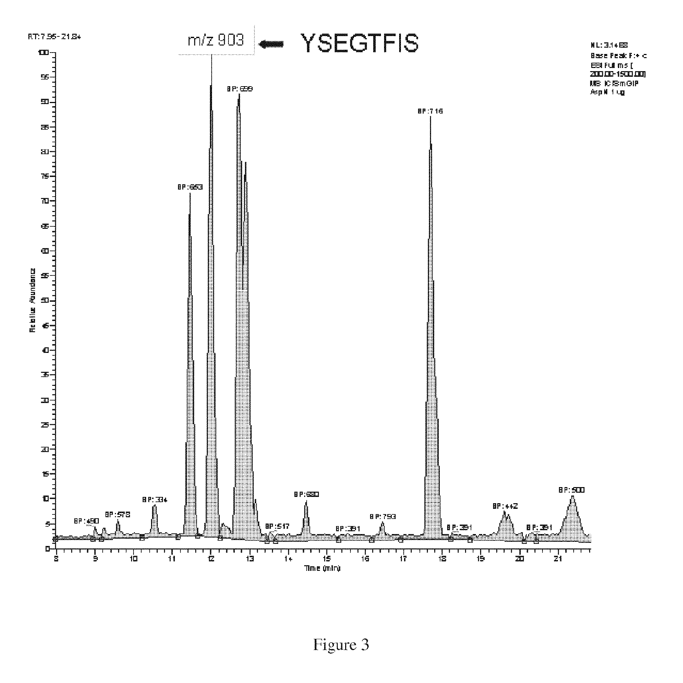

The results that the GIP analog was cleaved by Asp-N and analyzed are shown in Figure 3. One peak (m/z 903 ([M + H]⁺)) was observed. This peak was 2S-GIP₁₋₈ in which the N-terminal side of aspartic acid at position 9 of the active GIP analog was cleaved. 2S-GIP₁₋₈: Tyr-Ser-Glu-Gly-Thr-Phe-Ile-Ser [SEQ ID NO.: 5]

### <Example 2> Measuring method for GIP

### Method

An embodiment for measuring active GIP [SEQ ID NO.: 13], a glucagon-secretin family peptide, is shown. As a cleaving agent, formic acid was used. Active GIP₁₋₄₂ was dissolved with purified water to obtain a 100 µM solution. To 50 µL of this solution, 500 µL of 2% formic acid was added, and the solution was incubated at 108°C for 18 hours and the cleavage of active GIP₁₋₄₂ was confirmed with time. After that, each sample was analyzed by LC/MS (LCQ Deca XP Plus).

### Results

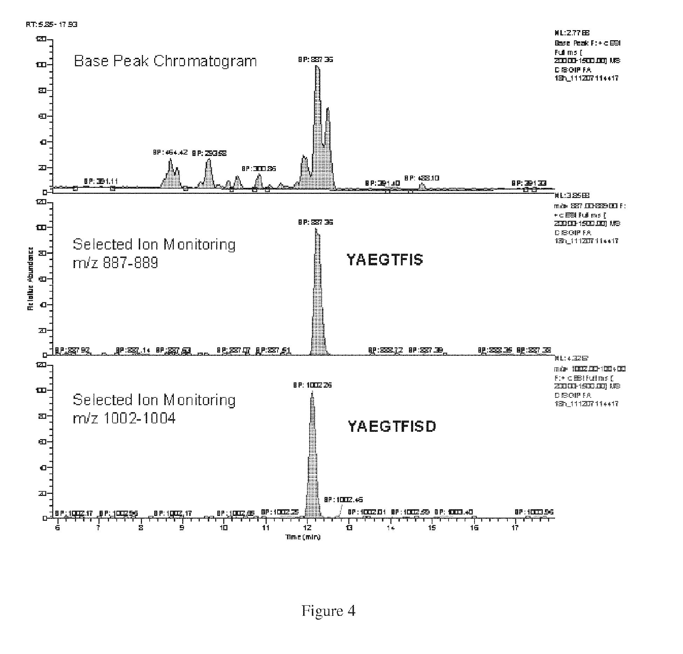

The results that active GIP₁₋₄₂ was cleaved by formic acid and analyzed are shown in Figure 4. Peaks of m/z 887 ([M + H]⁺) and m/z 1002 ([M + H]⁺) were observed. These two peaks were GIP₁₋₈ in which the N-terminal side of aspartic acid at position 9 of active GIP₁₋₄₂ was cleaved and GIP₁₋₉ in which the C-terminal side of aspartic acid at position 9 of active GIP₁₋₄₂ was cleaved.
GIP₁₋₈: Tyr-Ala-Glu-Gly-Thr-Phe-Ile-Ser [SEQ ID NO.: 1]
GIP₁₋₉: Tyr-Ala-Glu-Gly-Thr-Phe-Ile-Ser-Asp [SEQ ID NO.: 2]

### <Example 3> Measuring method for GIP

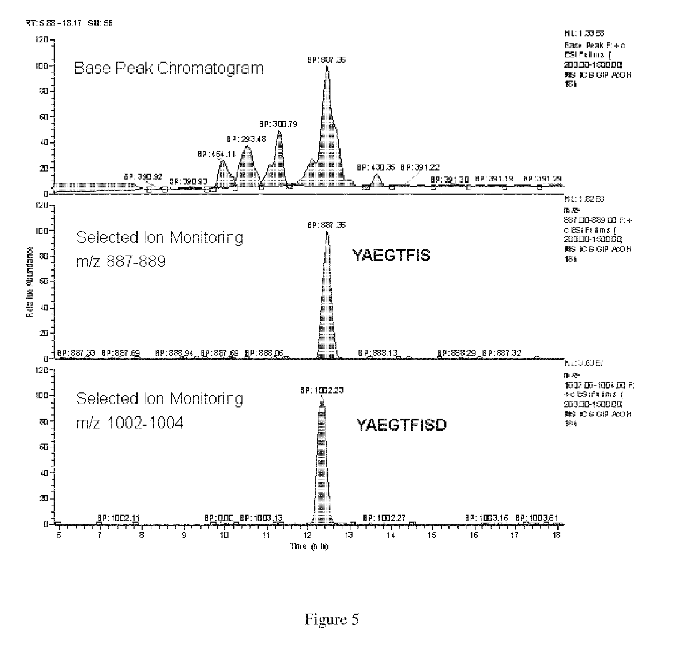

Another embodiment for measuring active GIP [SEQ ID NO.: 13], a glucagon-secretin family peptide, is shown. As a cleaving agent, acetic acid was used. The analysis was carried out by the same method as in Example 2 except that 500 µL of 2% acetic acid was added in place of 500 µL of 2% formic acid. The analysis results are shown in Figure 5. Peaks of GIP₁₋₈ [SEQ ID NO.: 1] in which the N-terminal side of aspartic acid at position 9 of active GIP₁₋₄₂ was cleaved and GIP₁₋₉ [SEQ ID NO.: 2] in which the C-terminal side of aspartic acid at position 9 of active GIP₁₋₄₂ was cleaved were observed.

### <Example 4> Measuring method for GIMP

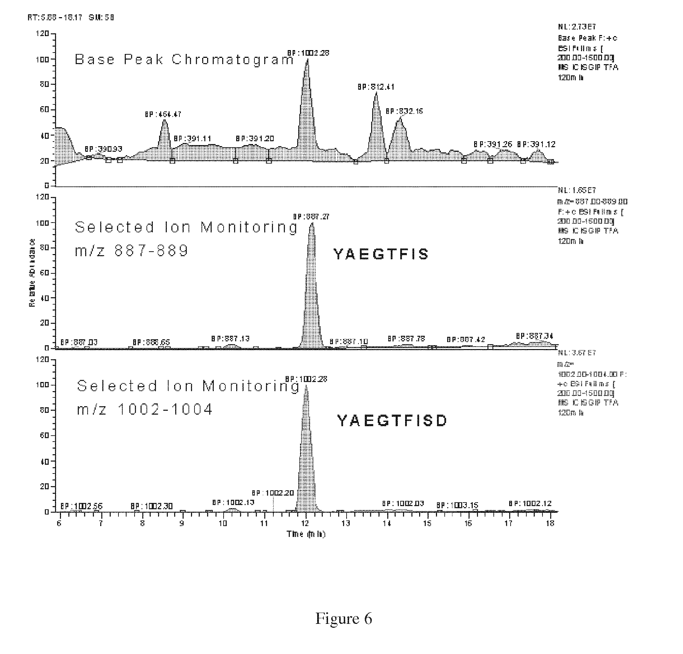

Another embodiment for measuring active GIP [SEQ ID NO.: 13], a glucagon-secretin family peptide, is shown. As a cleaving agent, trifluoroacetic acid was used. The analysis was carried out by the same method as in Example 2 except that 500 µL of 1% trifluoroacetic acid was added in place of 500 µL of 2% formic acid. The analysis results are shown in Figure 6. Peaks of GIP₁₋₈ [SEQ ID NO.: 1] in which the N-terminal side of aspartic acid at position 9 of active GIP₁₋₄₂ was cleaved and GIP₁₋₉ [SEQ ID NO.: 2] in which the C-terminal side of aspartic acid at position 9 of active GIP₁₋₄₂ was cleaved were observed.

### <Example 5> Measuring method for GLP-1

### Method

An embodiment for measuring GLP-1 (7-36) [SEQ ID NO.: 25], a glucagon-secretin family peptide, is shown. As a cleaving agent, ASP-N was used. The analysis was carried out by the same method as in Example 1 except that GLP-1 (7-36) was used in place of a GIP analog.

### Results

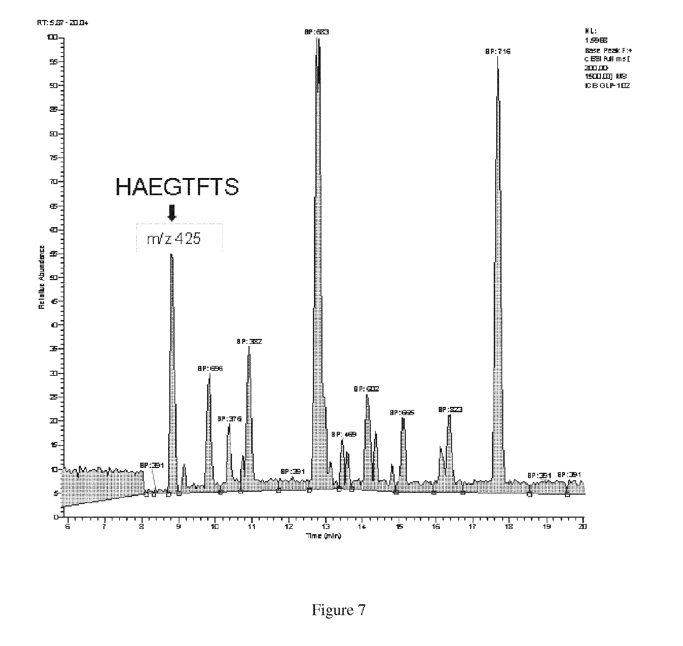

The analysis results are shown in Figure 7. A peak of GLP-1 (7-14) in which the N-terminal side of aspartic acid at position 15 of GLP-1 (7-36) was cleaved was observed (m/z 425 ([M + 2H]²⁺)).
GLP-1 (7-14): His-Ala-Glu-Gly-Thr-Phe-Thr-Ser [SEQ ID NO.: 6]

### <Example 6> Measuring method for glucagon

### Method

An embodiment for measuring glucagon [SEQ ID NO.: 69], a glucagon-secretin family peptide, is shown. As a cleaving agent, ASP-N was used. The analysis was carried out by the same method as in Example 1 except that glucagon was used in place of a GIP analog.

### Results

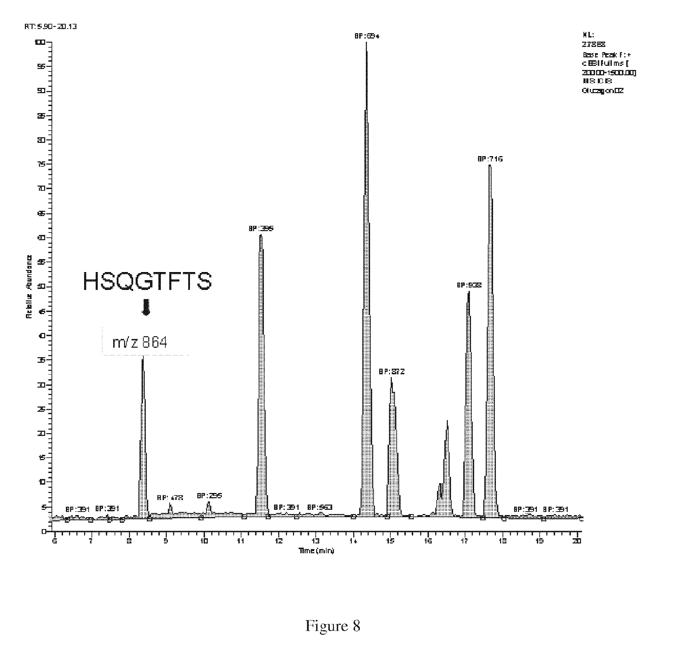

The analysis results are shown in Figure 8. A peak of Glucagon₁₋₈ in which the N-terminal side of aspartic acid at position 9 of glucagon was cleaved was observed (m/z 864 ([M+H]⁺)).

Glucagon₁₋₈: His-Ser-Gln-Gly-Thr-Phe-Thr-Ser [SEQ ID NO.: 11]

### <Example 7> Measuring method for a GLP-1 analog

### Method

An embodiment for measuring a GLP-1 analog (8S-GLP-1) [SEQ ID NO.: 31], a glucagon-secretin family peptide, is shown. This analog is a GLP-1 analog in which alanine at position 8 of GLP-1 (7-36) is substituted with serine. As a cleaving agent, ASP-N was used. The analysis was carried out by the same method as in Example 1 except that 5 nmol of a GLP-1 analog was used in place of 5 nmol of a GIP analog and the amount of Asp-N was changed from 1 µg to 2 µg.

### Results

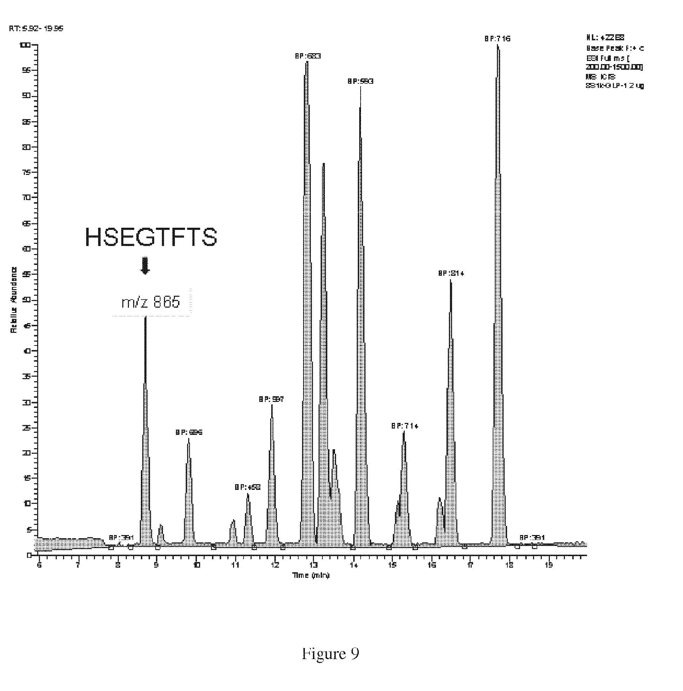

The analysis results are shown in Figure 9. A peak of 8S-GLP-1 (7-14) in which the N-terminal side of aspartic acid at position 14 of the GLP-1 analog was cleaved was observed (m/z 865 ([M + H]⁺)).
8S-GLP-1 (7-14): His-Ser-Glu-Gly-Thr-Phe-Thr-Ser [SEQ ID NO.: 10]

As shown in Examples 1 to 7, by cleaving the peptide bond of any aspartic acid selected from positions 1-14 of glucagon-secretin family peptides, peptide fragments of glucagon-secretin family peptides could be favorably analyzed regardless of types of glucagon-secretin family peptides.

### <Example 8> Quantitative method for active GIP

An embodiment for quantitating the concentration of active GIP [SEQ ID NO.: 13] in a sample using a calibration curve is shown.

### Method

### 1. Preparation of calibration curve samples

As an internal standard, the following internal standard was synthesized, in which Phe at position 6 of GIP was substituted with ¹³C₉,¹⁵N-Phe, a stable isotope-labeled amino acid. Calibration curve samples were prepared according to the following (1) to (3) procedures.

### Standard

Tyr-Ala-Glu-Gly-Thr-Phe-Ile-Ser-Asp-Tyr-Ser-Ile-Ala-Met-Asp-Lys-Ile-His-Gln-Gln-Asp-P he-Val-Asn-Trp-Leu-Leu-Ala-Gln-Lys-Gly-Lys-Lys-Asn-Asp-Trp-Lys-His-Asn-Ile-Thr-Gln [SEQ ID NO.: 13]

### Internal standard

Tyr-Ala-Glu-Gly-Thr-¹³C₉,¹⁵N-Phe-Ile-Ser-Asp-Tyr-Ser-Ile-Ala-Met-Asp-Lys-Ile-His-Gln-Gl n-Asp-Phe-Val-Asn-Trp-Leu-Leu-Ala-Gln-Lys-Gly-Lys-Lys-Asn-Asp-Trp-Lys-His-Asn-Ile-Thr-Gln (the amino acid sequence is the same as SEQ ID NO.: 13)
(1) Preparation of standard solutions of active GIP: active GIP₁₋₄₂ (purchased from Peptide Institute, Inc.) was dissolved with purified water to prepare a 100 µM solution. This solution was diluted to prepare standard solutions, each having 20.0 pM, 200 pM, 2.00 nM, 20.0 nM and 1.00 µM.
(2) Preparation of standard solutions of the internal standard: the above-mentioned synthesized internal standard was dissolved to prepare a 100 µM solution. This solution was diluted to prepare standard solutions of the internal standard, each having 2.00 nM, 20.0 nM and 1.00 µM.
(3) Preparation of calibration curve samples: in a 2 mL tube, 2 µL of DPP-4 inhibitor (Diprotin A, 0.3 M) was added, and 200 µL of human EDTA-added plasma treated with activated charcoal, a standard solution of active GIP and a standard solution of the internal standard were added thereto under ice-cold conditions to prepare a calibration curve sample. The percentages, in which the standard solution of active GIP and the standard solution of the internal standard are added, are shown in Table 2. DPP-4 inhibitor was added to inhibit decomposition of GIP₁₋₄₂ to GIP₃₋₄₂ or decomposition of GIP₁₋₈ to GIP₃₋₈ in the pretreatment process.

**Table 2**

| Calibration curve concentration (pM Plasma) | Concentration of standard solution of GIP (pM) | Addition amount of standard solution of GIP (µL) | Concentration of standard solution of internal standard substance(pM) | Addition amount of standard solution of internal standard substance(µL) |
|---|---|---|---|---|
| 1 | 20 | 10 | 2000 | 10 |
| 2 | 20 | 20 | 2000 | 10 |
| 5 | 20 | 50 | 2000 | 10 |
| 10 | 200 | 10 | 2000 | 10 |
| 20 | 200 | 20 | 2000 | 10 |
| 50 | 200 | 50 | 2000 | 10 |
| 100 | 2000 | 10 | 2000 | 10 |
| 200 | 2000 | 20 | 2000 | 10 |
| 500 | 2000 | 50 | 2000 | 10 |

### 2. Pretreatment

### (1) Deproteinization

To the above-mentioned calibration curve sample, 100 µL of a 180 mM solution of ammonium carbonate and 900 µL of ethanol were added, and the obtained mixture was stored on ice for 20 minutes. After centrifugation, supernatant was concentrated to dryness.

### (2) Cleavage of a glucagon-secretin family peptide in a sample

The above calibration curve sample after deproteinization was cleaved by a protease (ASP-N). To the above calibration curve sample after deproteinization, 100 µL of a 50 mM Tris/HCl and 2.5 mM ZnSO₄ solution (pH 8.0) were added, and further 8 µL of a 0.1 mg/mL Asp-N aqueous solution was added, and the obtained mixture was incubated at 37°C for 16 hours.

### (3) Solid phase extraction

Using a solid phase plate (manufactured by Waters; Oasis MAX 96-well µElution Plate), 300 µL of 5% ammonium water was added to the calibration curve sample obtained in the above-mentioned (2), and this was loaded on the solid phase plate subjected to conditioning. The solid phase plate was washed, followed by elution with 50 µL of 0.1% formic acid-75% acetonitrile. The eluate was transferred to a HPLC vial, and concentrated to dryness using a centrifugal evaporator, and further reconstituted with 20 µL of 0.1% TFA-10% acetonitrile.

### (4) Filtration through a filter

The sample after reconstituted was subjected to centrifugal filtration by a centrifugal filter unit (0.2 µm) to obtain a HPLC sample.

### 3. Measurement

In nanoFlow LC-MS/MS/MS, 5 µL of the above HPLC sample subjected to pretreatment was injected, and peptides derived from the standard and the internal standard contained in the HPLC sample were measured. The measuring conditions are shown in Table 3 and Table 4.

### (1) HPLC conditions

**[Table 3]**

| | |
|---|---|
| LC system | Ultimate™3000 nano-LC system |
| Trap column | C18, 5 µm, 100 Å 300 µm i.d. x 1 mm |
| Mobile phase for trap column | 0.1 % TFA-2 % Acetonitrile |
| Flow rate | 25 µL/min |
| Analytical column | C18, 3 µm, 100Å, 75 µm i.d. x 15 cm |
| Mobile phase A | 0.1% formic acid-2% methanol |
| Mobile phase B | 0.1% formic acid-95% methanol |
| Flow rate | 250 nL/min |

### (2) MS conditions

**[Table 4]**

| | |
|---|---|
| Mass spectrometer | QTRAP® 5500 |
| Ionization method | Nanospray ESI |
| Polarity | Positive |
| Detection mode | MRM3 (Linear ion trap mode) |
| Monitor ion of substance to be measured | m/z 887.4 → m/z 782.4 → m/z 764.2 |
| Monitor ion of internal standard substance | m/z 897.4 → m/z 792.4 → m/z 774.2 |

### Results

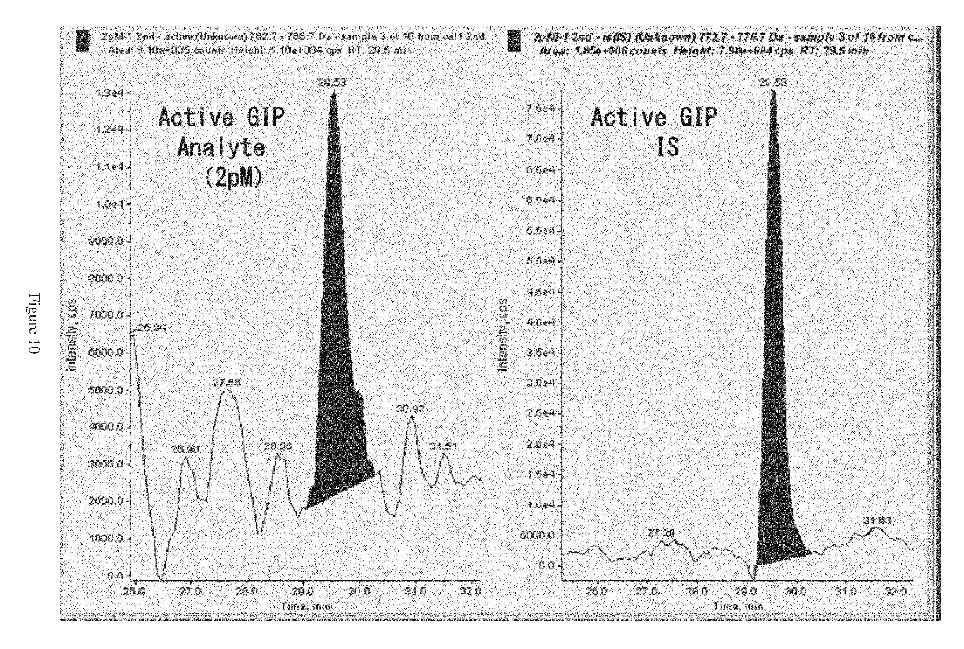

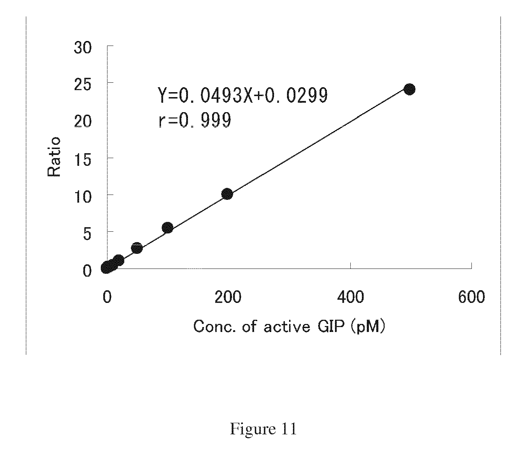

The calibration curve samples were measured, and the peptide fragment derived from active GIP (Tyr-Ala-Glu-Gly-Thr-Phe-Ile-Ser [SEQ ID NO.: 1]) and the peptide fragment derived from the internal standard (Tyr-Ala-Glu-Gly-Thr-¹³C₉,¹⁵N-Phe-Ile-Ser (the amino acid sequence is the same as SEQ ID NO.: 1)) could be detected as each individual peak (Figure 10). The concentration of active GIP and the peak area ratio of the two (the peak area of the peptide derived from active GIP/the peak area of the peptide derived from the internal standard) were subjected to linear regression by the linear least-squares method to calculate a slope and an intercept and a correlation coefficient (r). A calibration curve was made in a range of 1 pM to 500 pM. Consequently, the correlation coefficient (r) was 0.990 or more and the lower limit of quantification was 1 pM (Figure 11).

### <Example 9> Simultaneous (intra-day) reproducibility test of active GIP in human plasma

### Method

Active GIP in human plasma [SEQ ID NO.: 13] was quantitated in the same manner as in Example 8, and the obtained peak area ratio was applied to each calibration curve to calculate the concentration of each sample. The accuracy (Bias %) and precision (%CV) of the obtained measured values (n = 3) were calculated at each concentration. The criteria were that the accuracy be within ± 15% (the lower limit of quantification is within ± 20%), and the precision be within 15% (the lower limit of quantification is within 20%).

### Results

The results are shown in 5. Active GIP at each concentration satisfied the criteria for simultaneous reproducibility. This shows that reproducibility of measured values is good.

**[Table 5]**

| | Theoretical value (pM) | | | | | | | | |
|---|---|---|---|---|---|---|---|---|---|
| | 1 | 2 | 5 | 10 | 20 | 50 | 100 | 200 | 500 |
| Calibration curve 1 | 0.996 | 2.13 | 4.49 | 8.86 | 20.6 | 52.6 | 109 | 202 | 487 |
| Calibration curve 2 | 0.830 | 1.82 | 4.29 | 11.3 | 22.4 | 54.5 | 108 | 204 | 481 |
| Calibration curve 3 | 0.847 | 2.27 | 5.51 | 8.80 | 20.7 | 51.8 | 92.8 | 208 | 498 |
| Mean (pM) | 0.891 | 2.07 | 4.76 | 9.65 | 21.2 | 53.0 | 103 | 205 | 489 |
| S.D. | 0.091 | 0.23 | 0.65 | 1.43 | 1.0 | 1.4 | 9 | 3 | 9 |
| % CV | 10.3 | 11.1 | 13.7 | 14.8 | 4.8 | 2.6 | 8.8 | 1.5 | 1.8 |
| Bias % | -10.9 | 3.7 | -4.7 | -3.5 | 6.2 | 5.9 | 3.3 | 2.3 | -2.3 |

### <Example 10> Simultaneous quantitative method for active GIP and inactive GIP

### Method

### 1. Preparation of calibration curve samples

Measurement samples containing a standard and an internal standard were prepared in the same manner as in Example 8. As an internal standard, the following peptide was synthesized and added, in which Phe at position 6 of GIP was substituted with ¹³C₉,¹⁵N-Phe. An internal standard of inactive GIP is one in which the N-terminal two residues of the internal standard of active GIP are deleted.

### [Standard of active GIP]

Tyr-Ala-Glu-Gly-Thr-Phe-Ile-Ser-Asp-Tyr-Ser-Ile-Ala-Met-Asp-Lys-Ile-His-Gln-Gln-Asp-P he-Val-Asn-Trp-Leu-Leu-Ala-Gln-Lys-Gly-Lys-Lys-Asn-Asp-Trp-Lys-His-Asn-Ile-Thr-Gln

### [SEQ ID NO.: 13]

### [Standard of inactive GIP]

Glu-Gly-Thr-Phe-Ile-Ser-Asp-Tyr-Ser-Ile-Ala-Met-Asp-Lys-Ile-His-Gln-Gln-Asp-Phe-Val-A sn-Trp-Leu-Leu-Ala-Gln-Lys-Gly-Lys-Lys-Asn-Asp-Trp-Lys-His-Asn-Ile-Thr-Gln (GIP₃₋₄₂:

### [SEQ ID NO.: 14])

### [Internal standard of active GIP]

Tyr-Ala-Glu-Gly- Thr-¹³C₉,¹⁵N-Phe-Ile-Ser-Asp-Tyr-Ser-Ile-Ala-Met-Asp-Lys-Ile-His-Gln-Gl n-Asp-Phe-Val-Asn-Trp-Leu-Leu-Ala-Gln-Lys-Gly-Lys-Lys-Asn-Asp-Trp-Lys-His-Asn-Ile-Thr-Gln (the amino acid sequence is the same as SEQ ID NO.: 13)

### [Internal standard of inactive GIP]

Glu-Gly- Thr-¹³C₉,¹⁵N- Phe- Ile-Ser-Asp-Tyr-Ser-Ile-Ala-Met-Asp-Lys-Ile-His-Gln-Gln-Asp-P he-Val-Asn-Trp-Leu-Leu-Ala-Gln-Lys-Gly-Lys-Lys-Asn-Asp-Trp-Lys-His-Asn-Ile-Thr-Gln

### (the amino acid sequence is the same as SEQ ID NO.: 14)

(1) Preparation of standard solutions of active GIP: active GIP (purchased from Peptide Institute, Inc.) was dissolved by adding purified water to obtain a 100 µM solution. This solution was diluted with 50% ethanol to prepare standard solutions, each having 20.0 pM, 200 pM, 2.00 nM, 20.0 nM and 1.00 µM.
(2) Preparation of standard solutions of the internal standard for active GIP: the synthesized internal standard peptide was dissolved to prepare a 100 µM solution. This solution was diluted to prepare standard solutions, each having 1.00 nM, 10.0 nM and 1.00 µM.
(3) Preparation of standard solutions of inactive GIP: purchased inactive GIP was dissolved to prepare a 100 µM solution. This solution was diluted to prepare standard solutions, each having 20.0 pM, 200 pM, 2.00 nM, 20.0 nM and 1.00 µM.
(4) Preparation of standard solutions of the internal standard for inactive GIP: the synthesized internal standard peptide was dissolved with 50% ethanol to prepare a 100 µM solution. This solution was diluted with 50% ethanol to prepare standard solutions, each having 1.00 nM, 10.0 nM and 1.00 µM.
(5) Preparation of calibration curve samples: in 2 mL tube, 2 µL of DPP-4 inhibitor (Diprotin A, 0.3 M) was added, and 200 µL of human EDTA-added plasma treated with activated charcoal, a standard solution of active GIP, and a standard solution of the internal standard for active GIP, as well as a standard solution of inactive GIP, and a standard solution of the internal standard for inactive GIP were added thereto under ice-cold conditions as shown in Table 6 to prepare calibration curve samples.

**[Table 6]**

| Calibration curve concentration (pM Plasma) | Concentration of standard solution of active GIP (pM) | Addition amount of standard solution of active GIP (µL) | Concentration of standard solution of internal standard substance for active GIP (pM) | Addition amount of standard solution of internal standard substance for active GIP (µL) | Concentration of standard solution of inactive GIP (pM) | Addition amount of standard solution of inactive GIP (µL) | Concentration of standard solution of internal standard substance for inactive GIP (pM) | Addition amount of standard solution of internal standard substance for inactive GIP (µL) |
|---|---|---|---|---|---|---|---|---|
| 1 | 20 | 10 | 1000 | 10 | - | - | - | - |
| 2 | 20 | 20 | 1000 | 10 | - | - | - | - |
| 5 | 20 | 50 | 1000 | 10 | - | - | - | - |
| 10 | 200 | 10 | 1000 | 10 | 200 | 10 | 10000 | 40 |
| 20 | 200 | 20 | 1000 | 10 | 200 | 20 | 10000 | 40 |
| 50 | 200 | 50 | 1000 | 10 | 200 | 50 | 10000 | 40 |
| 100 | 2000 | 10 | 1000 | 10 | 2000 | 10 | 10000 | 40 |
| 200 | 2000 | 20 | 1000 | 10 | 2000 | 20 | 10000 | 40 |
| 500 | 2000 | 50 | 1000 | 10 | 2000 | 50 | 10000 | 40 |

### 2. Pretreatment

The pretreatment was carried out by the same method as in Example 8.

### 3. Measurement

The measurement was carried out in the same manner as in Example 8. The measuring conditions are shown in Table 7 and Table 8.

### (1) HPLC conditions

**[Table 7]**

| | |
|---|---|
| LC system | Ultimate™3000 nano-LC system |
| Trap column | C18, 5 µm, 100Å, 300 µm i.d. x 1 mm |
| Mobile phase for trap column | 0.1 % TFA-2 % Acetonitrile |
| Flow rate | 25 µL/min |
| Analytical column | C18, 3 µm, 100Å, 75 µm i.d. x 15cm |
| Mobile phase A | 0.1% formic acid-2% methanol |
| Mobile phase B | 0.1% formic acid-95% methanol |
| Flow rate | 250 nL/min |

### (2) MS conditions

**[Table 8]**

| | |
|---|---|
| Mass spectrometer | QTRAP® 5500 |
| Ionization method | Nanospray ESI |
| Polarity | Positive |
| | |

| Quantitative determination of active GIP | |
|---|---|
| Detection mode | MRM3 (Linear ion trap mode) |
| Monitor ion of substance to be measured | m/z 887.4 → m/z 782.4 → m/z 764.2 |
| Monitor ion of internal standard substance | m/z 897.4 → m/z 792.4 → m/z 774.2 |
| | |

| Quantitative determination of non-active GIP | |
|---|---|
| Detection mode | MRM |
| Monitor ion of substance to be measured | m/z 653.2 → m/z 288.1, 435.3, 548.3 |
| Monitor ion of internal standard substance | m/z 663.2 → m/z 288.1, 445.2, 558.3 |

### Results

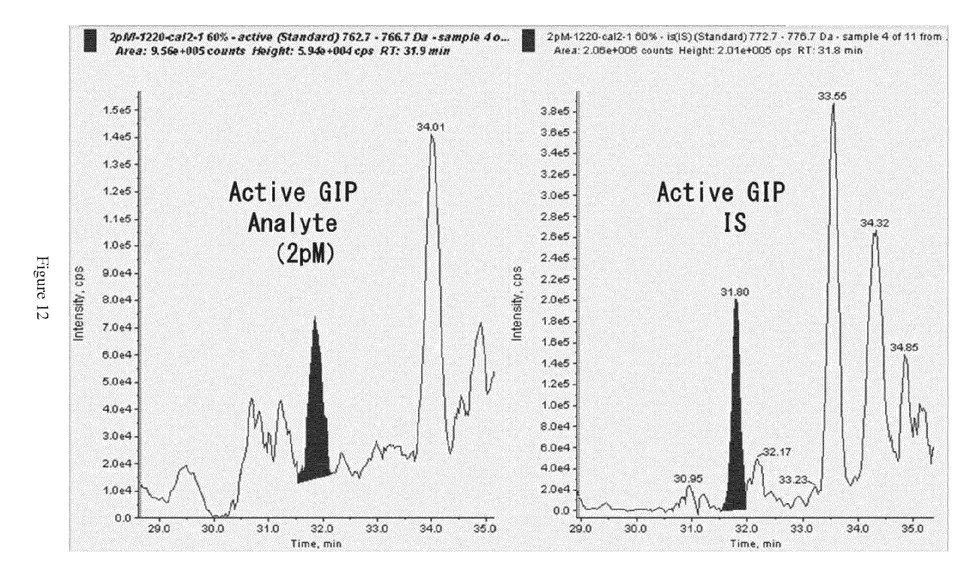

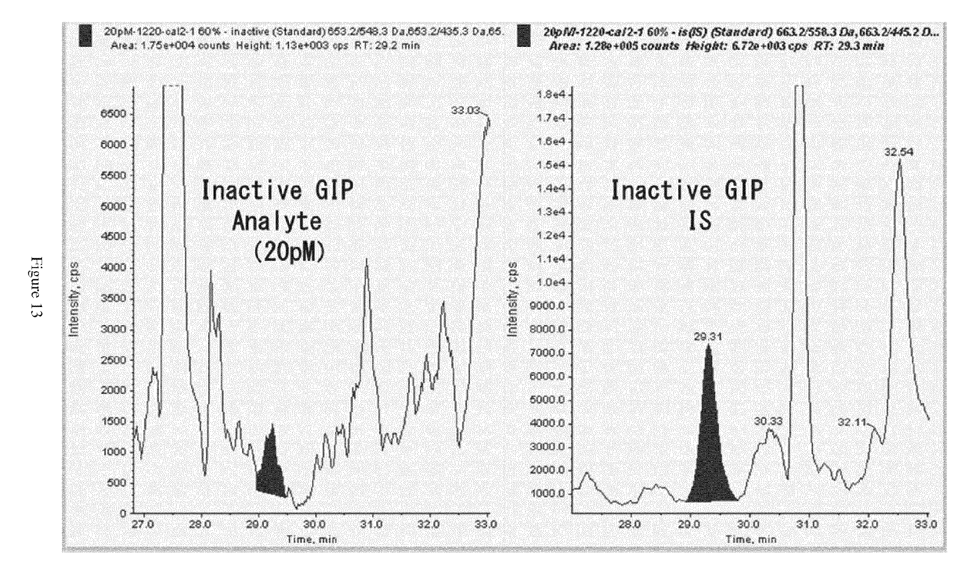

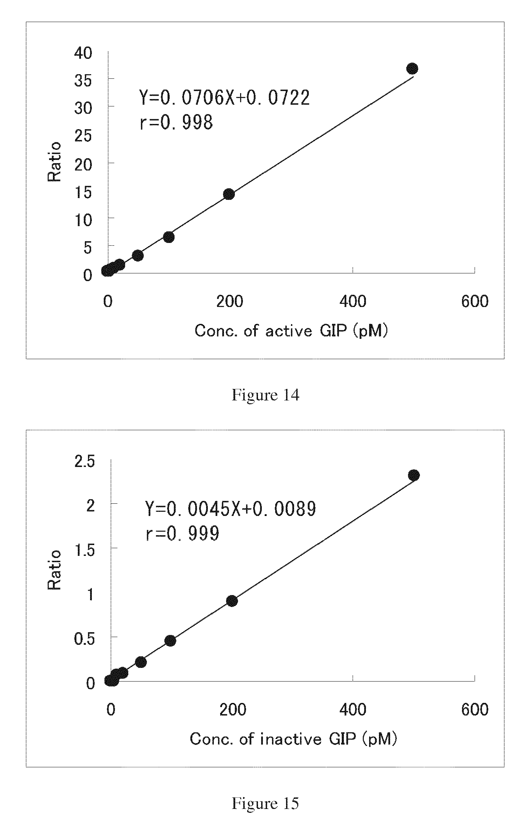

The calibration curve samples were measured, and the peptide fragment derived from active GIP (Tyr-Ala-Glu-Gly-Thr-Phe-Ile-Ser [SEQ ID NO.: 1]) and the peptide fragment derived from the internal standard for active GIP (Tyr-Ala-Glu-Gly-Thr-¹³C₉,¹⁵N-Phe-Ile-Ser (the amino acid sequence is the same as SEQ ID NO.: 1)) could be detected as each individual peak (Figure 12). The peptide fragment derived from inactive GIP (Glu-Gly-Thr-Phe-Ile-Ser [SEQ ID NO.: 3]) and the peptide fragment derived from the internal standard for inactive GIP (Glu-Gly-Thr-¹³C₉,¹⁵N-Phe-Ile-Ser (the amino acid sequence is the same as SEQ ID NO.: 3)) could be detected as each individual peak (Figure 13). The concentration of active GIP and the peak area ratio of the two (the peak area of the peptide derived from active GIP/the peak area of the peptide derived from the internal standard) were subjected to linear regression by the linear least-squares method to calculate a slope and an intercept and a correlation coefficient (r). A calibration curve was made in a range of 1 pM to 500 pM. Consequently, the correlation coefficient (r) was 0.990 or more with 1/x weighting (Figure 14). The concentration of inactive GIP and the peak area ratio of the two (the peak area of the peptide derived from inactive GIP/the peak area of the peptide derived from the internal standard for inactive GIP) were subjected to linear regression by the linear least-squares method to calculate a slope and an intercept and a correlation coefficient (r). A calibration curve was made in a range of 10 pM to 500 pM. Consequently, the correlation coefficient (r) was 0.990 or more with 1/x weighting (Figure 15).

### <Example 11> Simultaneous quantitative determination of active GIP and inactive GIP in diabetics

In 6 diabetics taking α-glucosidase inhibitors (50 mg, three times a day), active GIP [SEQ ID NO.: 13] and inactive GIP [SEQ ID NO.: 14] in plasma were simultaneously quantitated before eating and at one hour after eating.

### 1. Preparation of samples

### (1) Preparation of calibration curve samples

Samples were prepared as shown in Table 9 in the same manner as in Example 9.

**[Table 9]**

| Calibration curve concentration (pM Plasma) | Concentration of standard solution of active GIP (pM) | Addition amount of standard solution of active GIP (µL) | Concentration of standard solution of internal standard substance for active GIP (pM) | Addition amount of standard solution of internal standard substance for active GIP (µL) | concentration of standard solution of inactive GIP (pM) | Addition amount of standard solution of inactive GIP (µL) | concentration of standard solution of internal standard substance for inactive GIP (pM) | Addition amount of standard solution of internal standard substance for inactive GIP (µL) |
|---|---|---|---|---|---|---|---|---|
| 1 | 20 | 10 | 1000 | 20 | - | - | - | - |
| 2 | 20 | 20 | 1000 | 20 | - | - | - | - |
| 5 | 20 | 50 | 1000 | 20 | - | - | - | - |
| 10 | 200 | 10 | 1000 | 20 | 200 | 10 | 10000 | 20 |
| 20 | 200 | 20 | 1000 | 20 | 200 | 20 | 10000 | 20 |
| 50 | 200 | 50 | 1000 | 20 | 200 | 50 | 10000 | 20 |
| 100 | 2000 | 10 | 1000 | 20 | 2000 | 10 | 10000 | 20 |
| 200 | 2000 | 20 | 1000 | 20 | 2000 | 20 | 10000 | 20 |
| 500 | 2000 | 50 | 1000 | 20 | 2000 | 50 | 10000 | 20 |

### (2) Preparation of plasma samples from diabetics

Blood was collected from a patient taking an α-glucosidase inhibitor using a blood collection tube with a protein stabilizer (manufactured by Becton, Dickinson and Company) to obtain plasma. To 200 µL of this plasma, an internal standard for active GIP and an internal standard for inactive GIP were added in amounts equal to those when making the above-mentioned calibration curve.

### 2. Measurement

A sample subjected to pretreatment was measured in the same manner as in Example 8. The measuring conditions are shown in Table 10 and Table 12.

**[Table 10]**

| | |
|---|---|
| LC system | Ultimate™3000 nano-LC system |
| Trap column | C18, 5 µm, 100Å, 300 µm i.d. x 1 mm |
| Mobile phase for trap column | 0.1 % TFA-2 % Acetonitrile |
| Flow rate | 25 µL/min |
| Analytical column | C18, 3 µm, 100Å, 75 µm i.d. x 15 cm |
| Mobile phase A | 0.1% formic acid-2% methanol |
| Mobile phase B | 0.1% formic acid-95% methanol |
| Flow rate | 250 nL/min |

**[Table 11]**

| Gradient conditions | | |
|---|---|---|
| Time (min) | Mobile phase A (%) | Mobile phaseB (%) |
| 0 | 100 | 0 |
| 2 | 100 | 0 |
| 3 | 75 | 25 |
| 21.2 | 40 | 60 |
| 22 | 0 | 100 |
| 27 | 0 | 100 |
| 27.1 | 100 | 0 |
| 35 | 100 | 0 |

**[Table 12]**

| | |
|---|---|
| Mass spectrometer | QTRAP® 5500 |
| Ionization method | Nanospray ESI |
| Polarity | Positive |
| | |

| Quantitative determination of active GIP | |
|---|---|
| Detection mode | MRM3 (Linear ion trap mode) |
| Monitor ion of substance to be measured | m/z 887.4 → m/z 782.4 → m/z 764.2 |
| Monitor ion of internal standard substance | m/z 897.4 → m/z 792.4 → m/z 774.2 |
| Curtain Gas Setting (Nitrogen) | 10 psi |
| Collision Gas Setting (Nitrogen) | High |
| Gas 1 Setting (Zero Air) | 5.0 psi |
| Gas 2 Setting (Zero Air) | 0.0 psi |
| AF2 Setting | 0.12 |
| Interface Heater Temperature | 150 °C |
| | |

| Quantitative determination of inactive GIP | |
|---|---|
| Detection mode | MRM |
| Monitor ion of substance to be measured | m/z 653.2 → m/z 288.1, 435.3, 548.3 |
| Monitor ion of internal standard substance | m/z 663.2 → m/z 288.1, 445.2, 558.3 |
| Curtain Gas Setting (Nitrogen) | 10 psi |
| Collision Gas Setting (Nitrogen) | 12.0 |
| Gas 1 Setting (Zero Air) | 5.0 psi |
| Gas 2 Setting (Zero Air) | 0.0 psi |

### Results

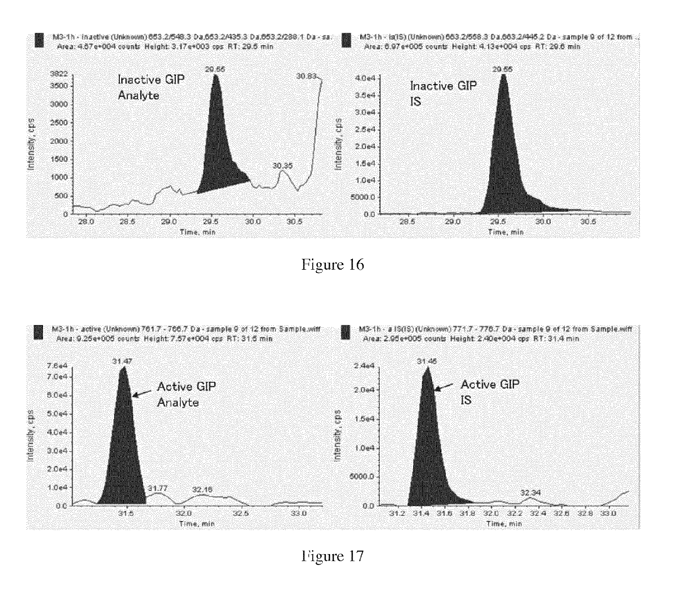

The plasma samples from diabetics were measured, and the peptide fragment derived from active GIP [SEQ ID NO.: 1] and the peptide fragment derived from the internal standard for active GIP (the amino acid sequence is the same as SEQ ID NO.: 1) could be detected as each individual peak (Figure 16). Similarly, inactive GIP could be also detected as each individual peak (Figure 17). The concentration of active GIP and the peak area ratio of the two (the peak area of the peptide derived from active GIP/the peak area of the peptide derived from the internal standard) were subjected to linear regression by the linear least-squares method to make a calibration curve with 1/x weighting in a range of 1 pM to 500 pM. The peak area ratio (the peak area of the peptide derived from active GIP/the peak area of the peptide derived from the internal standard) obtained from a plasma sample from a diabetic was applied to the calibration curve to calculate the concentration of active GIP in plasma. About inactive GIP, a calibration curve was made with 1/x weighting in a range of 10 pM to 500 pM in the same manner as for active GIP to calculate the concentration of inactive GIP in diabetic plasma. The results are shown in Table 13. The numerical values in the table show each average concentration in plasma ± standard deviation.

**[Table 13]**

| | Active GIP (pM) | Inactive GIP (pM) |
|---|---|---|
| Before eating | 19.2±8.9 | 58.4±33.2 |
| One hour after eating | 76.3±53.5 | 176±28 |

About other glucagon-secretin family peptides other than GIP, calibration curves are made in the same manner as in Examples 8 to 11, and using the calibration curves, glucagon-secretin family peptides in a sample can be quantitated. That is, a peptide to be quantitated is extracted together with an internal standard in which e.g. part of amino acids thereof is substituted with a stable isotope label. In the extraction process as shown in Examples 1 to 7, the peptide bond of any aspartic acid selected from positions 1-14 of glucagon-secretin family peptides is cleaved to obtain peptide fragments. Similarly to GIP, quantitative determination with high specificity can be carried out by setting mass spectrometry conditions depending on the peptide fragments. Further, as active GIP and inactive GIP can be simultaneously quantitated when extraction conditions are same, different glucagon-secretin family peptides can be simultaneously quantitated.

## Claims

1. An analysis method for a glucagon-secretin family peptide in a sample, the method comprising:
a cleavage step of cleaving a peptide bond of aspartic acid of a glucagon-secretin family peptide in a sample to obtain peptide fragments;
a separation/purification step of separating and purifying the peptide fragments cleaved in the cleavage step using liquid chromatography to select a peptide fragment, a substance to be measured, on an N-terminal glucagon-secretin family peptide; and
an analysis step of carrying out mass spectrometry to the peptide fragments separated and purified in the separation/purification step to produce the peptide fragment on the N-terminal glucagon-secretin family peptide.

2. The analysis method according to claim 1, wherein the peptide bond of aspartic acid is cleaved using a cleaving agent selected from a site specific protease or acid in the cleavage step.

3. The analysis method according to claim 2, wherein the cleaving agent is selected from the group consisting of endopeptidase ASP-N, formic acid, acetic acid, trifluoroacetic acid, propionic acid and a combination thereof.

4. The analysis method according to any of claims 1 to 3, wherein the liquid chromatography is liquid chromatography having a flow rate of 50 nL/min to 50 µL/min.

5. The analysis method according to any of claims 1 to 4, wherein the glucagon-secretin family peptide is selected from the group consisting of glucose-dependent insulinotropic polypeptide, glucagon-like peptide-1, glucagon-like peptide-2, glucagon and analogs thereof.

6. The analysis method according to any of claims 1 to 5, wherein the peptide fragment to be measured is selected by selecting precursor ion mass in the separation/purification step.

7. The analysis method according to any of claims 1 to 6, further comprising a step of solid-phase extraction of the peptide fragment in the separation/purification step.

8. The analysis method according to any of claims 1 to 7, wherein the separation/purification step and the analysis step are carried out by high performance liquid chromatography/mass spectrometry/mass spectrometry/mass spectrometry.

9. A quantitative method for a glucagon-secretin family peptide in a sample, wherein a calibration curve is made using an analysis method according to any of claims 1 to 8 and a glucagon-secretin family peptide in a sample is quantitated using the calibration curve.

10. The quantitative method according to claim 9, wherein two or more glucagon-secretin family peptides in a sample are distinguished and simultaneously quantitated by simultaneously measuring each peptide fragment of two or more glucagon-secretin family peptides.

11. The quantitative method according to claim 9 or 10, wherein active and inactive glucagon-secretin family peptides in a sample are distinguished and simultaneously quantitated by simultaneously measuring each peptide fragment of an active glucagon-secretin family peptide and an inactive glucagon-secretin family peptide.

12. The quantitative method according to any of claims 9 to 11, wherein a stable isotope-labeled internal standard is added to a sample to make the calibration curve.

13. The quantitative method according to claim 12, wherein quantitative determination is carried out using each peak area ratio of the peptide fragment and the internal standard peptide fragment.

14. The quantitative method according to claim 12 or 13, wherein the internal standard is a peptide in which one or more amino acids selected from positions 1-15 of a glucagon-secretin family peptide are substituted with stable isotope-labeled amino acid.

15. A quantitative kit for a glucagon-secretin family peptide, the kit comprising:
(a) a matrix for control,
(b) an internal standard or a solution thereof,
(c) glucagon-secretin family peptides or standard thereof, or solutions thereof,
(d) a cleaving agent, and
(e) a solid phase extraction plate.

16. The quantitative kit according to claim 15, wherein the cleaving agent is selected from the group consisting of endopeptidase ASP-N, formic acid, acetic acid, trifluoroacetic acid, propionic acid and a combination thereof.

17. The quantitative kit according to claim 15 or 16, wherein the glucagon-secretin family peptide is selected from the group consisting of glucose-dependent insulinotropic polypeptide, glucagon-like peptide-1, glucagon-like peptide-2, glucagon and analogs thereof.


SEQUENCE LISTING

<110> SANWA KAGAKU KENKYUSHO Co., LTD.

<120> An analysis method

<130> JTL/FP6915938

<140> EP 12755365.9
<141> 2012-03-07

<150> PCT/JP2012/055873
<151> 2012-03-07

<150> JP2011-049726
<151> 2011-03-08

<160> 113

<210> 1
<211> 8
<212> PRT
<213> Artificial

<220>
<223> GIP(1-8)

<400> 1

Tyr Ala Glu Gly Thr Phe Ile Ser
1               5

<210> 2
<211> 9
<212> PRT
<213> Artificial

<220>
<223> GIP(1-9)

<400> 2

Tyr Ala Glu Gly Thr Phe Ile Ser Asp
1               5

<210> 3

<211>  6
<212>  PRT
<213>  Artificial

<220>
<223>  GIP(3-8)

<400>  3

Glu Gly Thr Phe Ile Ser
1               5

<210>  4
<211>  7
<212>  PRT
<213>  Artificial

<220>
<223>  GIP(3-9)

<400>  4

Glu Gly Thr Phe Ile Ser Asp
1               5

<210>  5
<211>  8
<212>  PRT
<213>  Artificial

<220>
<223>  2S-GIP(1-8)

<400>  5

Tyr Ser Glu Gly Thr Phe Ile Ser
1               5

<210>  6
<211>  8
<212>  PRT
<213>  Artificial

<220>
<223>  GLP-1(7-14)

<400>   6

His Ala Glu Gly Thr Phe Thr Ser
1               5

<210>   7
<211>   9
<212>   PRT
<213>   Artificial

<220>
<223>   GLP-1(7-15)

<400>   7

His Ala Glu Gly Thr Phe Thr Ser Asp
1               5

<210>   8
<211>   6
<212>   PRT
<213>   Artificial

<220>
<223>   GLP-1(9-14)

<400>   8

Glu Gly Thr Phe Thr Ser
1               5

<210>   9
<211>   7
<212>   PRT
<213>   Artificial

<220>
<223>   GLP-1(9-15)

<400>   9

Glu Gly Thr Phe Thr Ser Asp
1               5

<210>  10
<211>  8
<212>  PRT
<213>  Artificial

<220>
<223>  8S-GLP-1(7-14)

<400>  10

His Ser Glu Gly Thr Phe Thr Ser
1               5



<210>  11
<211>  8
<212>  PRT
<213>  Artificial

<220>
<223>  Glocagon(1-8)

<400>  11

His Ser Gln Gly Thr Phe Thr Ser
1               5



<210>  12
<211>  9
<212>  PRT
<213>  Artificial

<220>
<223>  Glocagon(1-9)

<400>  12

His Ser Gln Gly Thr Phe Thr Ser Asp
1               5



<210>  13
<211>  42
<212>  PRT
<213>  Homo sapiens

<220>
<223>   GIP

<400>   13

Tyr Ala Glu Gly Thr Phe Ile Ser Asp Tyr Ser Ile Ala Met Asp Lys
1               5                   10                  15

Ile His Gln Gln Asp Phe Val Asn Trp Leu Leu Ala Gln Lys Gly Lys
            20                  25                  30

Lys Asn Asp Trp Lys His Asn Ile Thr Gln
        35                  40

<210>   14
<211>   40
<212>   PRT
<213>   Homo sapiens

<220>
<223>   GIP(3-42)

<400>   14

Glu Gly Thr Phe Ile Ser Asp Tyr Ser Ile Ala Met Asp Lys Ile His
1               5                   10                  15

Gln Gln Asp Phe Val Asn Trp Leu Leu Ala Gln Lys Gly Lys Lys Asn
            20                  25                  30

Asp Trp Lys His Asn Ile Thr Gln
        35                  40

<210>   15
<211>   42
<212>   PRT
<213>   Artificial

<220>
<223>   2G-GIP

<400>   15

Tyr Gly Glu Gly Thr Phe Ile Ser Asp Tyr Ser Ile Ala Met Asp Lys
1               5                   10                  15

Ile His Gln Gln Asp Phe Val Asn Trp Leu Leu Ala Gln Lys Gly Lys
            20                  25                  30

Lys Asn Asp Trp Lys His Asn Ile Thr Gln
        35                  40

<210>   16
<211>   42
<212>   PRT
<213>   Artificial

<220>
<223>   D2A-GIP

<220>
<221>   misc_feature
<222>   (2)..(2)
<223>   Xaa is (D)-Ala

<400>   16

Tyr Xaa Glu Gly Thr Phe Ile Ser Asp Tyr Ser Ile Ala Met Asp Lys
1               5                   10                  15

Ile His Gln Gln Asp Phe Val Asn Trp Leu Leu Ala Gln Lys Gly Lys
            20                  25                  30

Lys Asn Asp Trp Lys His Asn Ile Thr Gln
        35                  40

<210>   17
<211>   42
<212>   PRT
<213>   Artificial

<220>
<223>   2Aib-GIP

<220>
<221>  misc_feature
<222>  (2)..(2)
<223>  Xaa is Aib

<400>  17

Tyr Xaa Glu Gly Thr Phe Ile Ser Asp Tyr Ser Ile Ala Met Asp Lys
1               5                   10                  15

Ile His Gln Gln Asp Phe Val Asn Trp Leu Leu Ala Gln Lys Gly Lys
            20                  25                  30

Lys Asn Asp Trp Lys His Asn Ile Thr Gln
        35                  40

<210>  18
<211>  42
<212>  PRT
<213>  Artificial

<220>
<223>  2PS-GIP

<220>
<221>  misc_feature
<222>  (2)..(2)
<223>  Xaa is Phosphoserine

<400>  18

Tyr Xaa Glu Gly Thr Phe Ile Ser Asp Tyr Ser Ile Ala Met Asp Lys
1               5                   10                  15

Ile His Gln Gln Asp Phe Val Asn Trp Leu Leu Ala Gln Lys Gly Lys
            20                  25                  30

Lys Asn Asp Trp Lys His Asn Ile Thr Gln
        35                  40

<210>  19
<211>  42
<212>  PRT
<213>  Artificial

<220>
<223>  2Sar-GIP

<220>
<221>  misc_feature
<222>  (2)..(2)
<223>  Xaa is MeGly(sarcosine)

<400>  19

Tyr Xaa Glu Gly Thr Phe Ile Ser Asp Tyr Ser Ile Ala Met Asp Lys
1               5                   10                  15

Ile His Gln Gln Asp Phe Val Asn Trp Leu Leu Ala Gln Lys Gly Lys
            20                  25                  30

Lys Asn Asp Trp Lys His Asn Ile Thr Gln
        35                  40

<210>  20
<211>  42
<212>  PRT
<213>  Artificial

<220>
<223>  3P-GIP

<400>  20

Tyr Ala Pro Gly Thr Phe Ile Ser Asp Tyr Ser Ile Ala Met Asp Lys
1               5                   10                  15

Ile His Gln Gln Asp Phe Val Asn Trp Leu Leu Ala Gln Lys Gly Lys
            20                  25                  30

Lys Asn Asp Trp Lys His Asn Ile Thr Gln

35                    40

<210>  21
<211>  42
<212>  PRT
<213>  Artificial

<220>
<223>  3Hyp-GIP

<220>
<221>  misc_feature
<222>  (3)..(3)
<223>  Xaa is Hydroxyproline

<400>  21

Tyr Ala Xaa Gly Thr Phe Ile Ser Asp Tyr Ser Ile Ala Met Asp Lys
1               5                   10                  15

Ile His Gln Gln Asp Phe Val Asn Trp Leu Leu Ala Gln Lys Gly Lys
            20                  25                  30

Lys Asn Asp Trp Lys His Asn Ile Thr Gln
        35                  40

<210>  22
<211>  42
<212>  PRT
<213>  Artificial

<220>
<223>  3K-GIP

<400>  22

Tyr Ala Lys Gly Thr Phe Ile Ser Asp Tyr Ser Ile Ala Met Asp Lys
1               5                   10                  15

Ile His Gln Gln Asp Phe Val Asn Trp Leu Leu Ala Gln Lys Gly Lys
            20                  25                  30

Lys Asn Asp Trp Lys His Asn Ile Thr Gln
            35                          40

<210>   23
<211>   42
<212>   PRT
<213>   Artificial

<220>
<223>   3Y-GIP

<400>   23

Tyr Ala Tyr Gly Thr Phe Ile Ser Asp Tyr Ser Ile Ala Met Asp Lys
1                   5                        10                       15

Ile His Gln Gln Asp Phe Val Asn Trp Leu Leu Ala Gln Lys Gly Lys
                20                       25                       30

Lys Asn Asp Trp Lys His Asn Ile Thr Gln
            35                          40

<210>   24
<211>   42
<212>   PRT
<213>   Artificial

<220>
<223>   3F-GIP

<400>   24

Tyr Ala Phe Gly Thr Phe Ile Ser Asp Tyr Ser Ile Ala Met Asp Lys
1                   5                        10                       15

Ile His Gln Gln Asp Phe Val Asn Trp Leu Leu Ala Gln Lys Gly Lys
                20                       25                       30

Lys Asn Asp Trp Lys His Asn Ile Thr Gln
            35                          40

<210>  25
<211>  30
<212>  PRT
<213>  Homo sapiens

<220>
<223>  GLP-1(7-36)

<400>  25

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30

<210>  26
<211>  31
<212>  PRT
<213>  Homo sapiens

<220>
<223>  GLP-1(7-37)

<400>  26

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210>  27
<211>  28
<212>  PRT
<213>  Artificial

<220>
<223>  GLP-1(7-34)

<400>  27

His Ala Glu Gly Thr Phe Thr Ser Asp Val Ser Ser Tyr Leu Glu Gly

1               5                   10                  15

Gln Ala Ala Lys Glu Phe Ile Ala Trp Leu Val Lys
            20                  25

<210>  28
<211>  29
<212>  PRT
<213>  Artificial

<220>
<223>  GLP-1(7-35)

<400>  28

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly
            20                  25

<210>  29
<211>  28
<212>  PRT
<213>  Homo sapiens

<220>
<223>  GLP-1(9-36)

<400>  29

Glu Gly Thr Phe Thr Ser Asp Val Ser Ser Tyr Leu Glu Gly Gln Ala
1               5                   10                  15

Ala Lys Glu Phe Ile Ala Trp Leu Val Lys Gly Arg
            20                  25

<210>  30
<211>  29
<212>  PRT
<213>  Homp sapiens

<220>
<223>   GLP-1(9-37)

<400>   30

Glu Gly Thr Phe Thr Ser Asp Val Ser Ser Tyr Leu Glu Gly Gln Ala
1               5                   10                  15

Ala Lys Glu Phe Ile Ala Trp Leu Val Lys Gly Arg Gly
            20                  25

<210>   31
<211>   31
<212>   PRT
<213>   Artificial

<220>
<223>   8S-GLP-1(7-37)

<400>   31

His Ser Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210>   32
<211>   31
<212>   PRT
<213>   Artificial

<220>
<223>   8G-GLP-1(7-37)

<400>   32

His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210>  33
<211>  31
<212>  PRT
<213>  Artificial

<220>
<223>  8V-GLP-1(7-37)

<400>  33

His Val Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210>  34
<211>  31
<212>  PRT
<213>  Artificial

<220>
<223>  22E-GLP-1(7-37)

<400>  34

His Ala Glu Gly Thr Phe Thr Ser Asp Val Ser Ser Tyr Leu Glu Glu
1               5                   10                  15

Gln Ala Ala Lys Glu Phe Ile Ala Trp Leu Val Lys Gly Arg Gly
            20                  25                  30

<210>  35
<211>  31
<212>  PRT
<213>  Artificial

<220>
<223>  22K-GLP-1(7-37)

<400>  35

His Ala Glu Gly Thr Phe Thr Ser Asp Val Ser Ser Tyr Leu Glu Lys

1               5                   10                  15

Gln Ala Ala Lys Glu Phe Ile Ala Trp Leu Val Lys Gly Arg Gly
            20                  25                  30

<210>  36
<211>  31
<212>  PRT
<213>  Artificial

<220>
<223>  8V,22E-GLP-1(7-37)

<400>  36

His Val Glu Gly Thr Phe Thr Ser Asp Val Ser Ser Tyr Leu Glu Glu
1               5                   10                  15

Gln Ala Ala Lys Glu Phe Ile Ala Trp Leu Val Lys Gly Arg Gly
            20                  25                  30

<210>  37
<211>  31
<212>  PRT
<213>  Artificial

<220>
<223>  8V,22K-GLP-1(7-37)

<400>  37

His Val Glu Gly Thr Phe Thr Ser Asp Val Ser Ser Tyr Leu Glu Lys
1               5                   10                  15

Gln Ala Ala Lys Glu Phe Ile Ala Trp Leu Val Lys Gly Arg Gly
            20                  25                  30

<210>  38
<211>  31
<212>  PRT
<213>  Artificial

<220>
<223>   8G,22E-GLP-1(7-37)

<400>   38

His Gly Glu Gly Thr Phe Thr Ser Asp Val Ser Ser Tyr Leu Glu Glu
1               5                   10                  15

Gln Ala Ala Lys Glu Phe Ile Ala Trp Leu Val Lys Gly Arg Gly
            20                  25                  30

<210>   39
<211>   31
<212>   PRT
<213>   Artificial

<220>
<223>   8G,22K-GLP-1(7-37)

<400>   39

His Gly Glu Gly Thr Phe Thr Ser Asp Val Ser Ser Tyr Leu Glu Lys
1               5                   10                  15

Gln Ala Ala Lys Glu Phe Ile Ala Trp Leu Val Lys Gly Arg Gly
            20                  25                  30

<210>   40
<211>   31
<212>   PRT
<213>   Artificial

<220>
<223>   8V,30E-GLP-1(7-37)

<400>   40

His Val Glu Gly Thr Phe Thr Ser Asp Val Ser Ser Tyr Leu Glu Gly
1               5                   10                  15

Gln Ala Ala Lys Glu Phe Ile Glu Trp Leu Val Lys Gly Arg Gly
            20                  25                  30

<210>  41
<211>  31
<212>  PRT
<213>  Artificial

<220>
<223>  8G,30E-GLP-1(7-37)

<400>  41

His Gly Glu Gly Thr Phe Thr Ser Asp Val Ser Ser Tyr Leu Glu Gly
1               5                   10                  15

Gln Ala Ala Lys Glu Phe Ile Glu Trp Leu Val Lys Gly Arg Gly
            20                  25                  30

<210>  42
<211>  31
<212>  PRT
<213>  Artificial

<220>
<223>  8V,37H-GLP-1(7-37)

<400>  42

His Val Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg His
            20                  25                  30

<210>  43
<211>  31
<212>  PRT
<213>  Artificial

<220>
<223>  8G,37H-GLP-1(7-37)

<400>  43

His Gly Glu Gly Thr Phe Thr Ser Asp Val Ser Ser Tyr Leu Glu Gly

1               5                   10                  15

Gln Ala Ala Lys Glu Phe Ile Ala Trp Leu Val Lys Gly Arg His
            20                  25                  30

<210>  44
<211>  31
<212>  PRT
<213>  Artificial

<220>
<223>  34R-GLP-1(7-37)

<400>  44

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Arg Gly Arg Gly
            20                  25                  30

<210>  45
<211>  31
<212>  PRT
<213>  Artificial

<220>
<223>  18K-GLP-1(7-37)

<400>  45

His Ala Glu Gly Thr Phe Thr Ser Asp Val Ser Lys Tyr Leu Glu Gly
1               5                   10                  15

Gln Ala Ala Lys Glu Phe Ile Ala Trp Leu Val Lys Gly Arg Gly
            20                  25                  30

<210>  46
<211>  31
<212>  PRT
<213>  Artificial

<220>
<223>   8G,22E,36G-GLP-1(7-37)

<400>   46

His Gly Glu Gly Thr Phe Thr Ser Asp Val Ser Ser Tyr Leu Glu Glu
1               5                   10                  15

Gln Ala Ala Lys Glu Phe Ile Ala Trp Leu Val Lys Gly Gly Gly
            20                  25                  30

<210>   47
<211>   31
<212>   PRT
<213>   Artificial

<220>
<223>   8Aib,22Aib-GLP-1(7-37)

<220>
<221>   misc_feature
<222>   (2)..(2)
<223>   Xaa is Aib

<220>
<221>   misc_feature
<222>   (16)..(16)
<223>   Xaa is Aib

<400>   47

His Xaa Glu Gly Thr Phe Thr Ser Asp Val Ser Ser Tyr Leu Glu Xaa
1               5                   10                  15

Gln Ala Ala Lys Glu Phe Ile Ala Trp Leu Val Lys Gly Arg Gly
            20                  25                  30

<210>   48
<211>   31
<212>   PRT
<213>   Artificial

<220>

<223>   8Aib,35Aib-GLP-1(7-37)

<220>
<221>   misc_feature
<222>   (2)..(2)
<223>   Xaa is Aib

<220>
<221>   misc_feature
<222>   (29)..(29)
<223>   Xaa is Aib

<400>   48

His Xaa Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa Arg Gly
            20                  25                  30

<210>   49
<211>   31
<212>   PRT
<213>   Artificial

<220>
<223>   8Aib,22Aib,35Aib-GLP-1(7-37)

<220>
<221>   misc_feature
<222>   (2)..(2)
<223>   Xaa is Aib

<220>
<221>   misc_feature
<222>   (16)..(16)
<223>   Xaa is Aib

<220>
<221>   misc_feature
<222>   (29)..(29)
<223>   Xaa is Aib

<400>   49

His Xaa Glu Gly Thr Phe Thr Ser Asp Val Ser Ser Tyr Leu Glu Xaa
1               5                   10                  15

Gln Ala Ala Lys Glu Phe Ile Ala Trp Leu Val Lys Xaa Arg Gly
            20                  25                  30

<210>  50
<211>  31
<212>  PRT
<213>  Artificial

<220>
<223>  22E,23E-GLP-1(7-37)

<400>  50

His Ala Glu Gly Thr Phe Thr Ser Asp Val Ser Ser Tyr Leu Glu Glu
1               5                   10                  15

Glu Ala Ala Lys Glu Phe Ile Ala Trp Leu Val Lys Gly Arg Gly
            20                  25                  30

<210>  51
<211>  31
<212>  PRT
<213>  Artificial

<220>
<223>  8G,22E,23E-GLP-1(7-37)

<400>  51

His Gly Glu Gly Thr Phe Thr Ser Asp Val Ser Ser Tyr Leu Glu Glu
1               5                   10                  15

Glu Ala Ala Lys Glu Phe Ile Ala Trp Leu Val Lys Gly Arg Gly
            20                  25                  30

<210>  52
<211>  31
<212>  PRT

<213>   Artificial

<220>
<223>   8V,22E,23E-GLP-1(7-37)

<400>   52

His Val Glu Gly Thr Phe Thr Ser Asp Val Ser Ser Tyr Leu Glu Glu
1               5                   10                  15

Glu Ala Ala Lys Glu Phe Ile Ala Trp Leu Val Lys Gly Arg Gly
            20                  25                  30

<210>   53
<211>   31
<212>   PRT
<213>   Artificial

<220>
<223>   8V,22E,25V-GLP-1(7-37)

<400>   53

His Val Glu Gly Thr Phe Thr Ser Asp Val Ser Ser Tyr Leu Glu Glu
1               5                   10                  15

Gln Ala Val Lys Glu Phe Ile Ala Trp Leu Val Lys Gly Arg Gly
            20                  25                  30

<210>   54
<211>   31
<212>   PRT
<213>   Artificial

<220>
<223>   8V,22E,33I-GLP-1(7-37)

<400>   54

His Val Glu Gly Thr Phe Thr Ser Asp Val Ser Ser Tyr Leu Glu Glu
1               5                   10                  15

Gln Ala Ala Lys Glu Phe Ile Ala Trp Leu Ile Lys Gly Arg Gly

            20                  25                  30

<210> 55
<211> 31
<212> PRT
<213> Artificial

<220>
<223> 8V,22E,25V,33I-GLP-1(7-37)

<400> 55

His Val Glu Gly Thr Phe Thr Ser Asp Val Ser Ser Tyr Leu Glu Glu
1               5                   10                  15

Gln Ala Val Lys Glu Phe Ile Ala Trp Leu Ile Lys Gly Arg Gly
            20                  25                  30

<210> 56
<211> 39
<212> PRT
<213> Heloderma horridum

<220>
<223> Exending-3

<400> 56

His Ser Asp Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35

<210> 57
<211> 39
<212> PRT
<213> Heloderma suspectum

<220>
<223>   Exending-4

<400>   57

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35

<210>   58
<211>   44
<212>   PRT
<213>   Artificial

<220>
<223>   Exendin-4-KKKKK

<400>   58

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser Lys Lys Lys Lys Lys
        35                  40

<210>   59
<211>   45
<212>   PRT
<213>   Artificial

<220>
<223>   Exendin-4-KKKKKK

<400>   59

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser Lys Lys Lys Lys Lys Lys
        35                  40                  45

<210>   60
<211>   30
<212>   PRT
<213>   Artificial

<220>
<223>   Exendin-4(1-30)

<400>   60

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly
            20                  25                  30

<210>   61
<211>   28
<212>   PRT
<213>   Artificial

<220>
<223>   Exendin-4(1-28)

<400>   61

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn
            20                  25

<210>  62
<211>  39
<212>  PRT
<213>  Artificial

<220>
<223>  14L,25F-Exendin-4

<400>  62

His Gly Glu Gly Thr Phe Thr Ser Asp Leu Ser Lys Gln Leu Glu Glu
1               5                   10                  15

Glu Ala Val Arg Leu Phe Ile Glu Phe Leu Lys Asn Gly Gly Pro Ser
            20                  25                  30

Ser Gly Ala Pro Pro Pro Ser
        35

<210>  63
<211>  28
<212>  PRT
<213>  Artificial

<220>
<223>  14L,25F-Exendin-4(1-28)

<400>  63

His Gly Glu Gly Thr Phe Thr Ser Asp Leu Ser Lys Gln Leu Glu Glu
1               5                   10                  15

Glu Ala Val Arg Leu Phe Ile Glu Phe Leu Lys Asn
            20                  25

<210>  64
<211>  33
<212>  PRT
<213>  Homo sapiens

<220>
<223>  GLP-2

<400>  64

His Ala Asp Gly Ser Phe Ser Asp Glu Met Asn Thr Ile Leu Asp Asn
1               5                   10                  15

Leu Ala Ala Arg Asp Phe Ile Asn Trp Leu Ile Gln Thr Lys Ile Thr
            20                  25                  30

Asp

<210>  65
<211>  31
<212>  PRT
<213>  Homo sapiens

<220>
<223>  GLP-2(3-33)

<400>  65

Asp Gly Ser Phe Ser Asp Glu Met Asn Thr Ile Leu Asp Asn Leu Ala
1               5                   10                  15

Ala Arg Asp Phe Ile Asn Trp Leu Ile Gln Thr Lys Ile Thr Asp
            20                  25                  30

<210>  66
<211>  33
<212>  PRT
<213>  Artificial

<220>
<223>  2S-GLP-2

<400>  66

His Ser Asp Gly Ser Phe Ser Asp Glu Met Asn Thr Ile Leu Asp Asn
1               5                   10                  15

Leu Ala Ala Arg Asp Phe Ile Asn Trp Leu Ile Gln Thr Lys Ile Thr

20                  25                  30

Asp

<210>  67
<211>  33
<212>  PRT
<213>  Artificial

<220>
<223>  2G-GLP-2

<400>  67

His Gly Asp Gly Ser Phe Ser Asp Glu Met Asn Thr Ile Leu Asp Asn
1               5                   10                  15

Leu Ala Ala Arg Asp Phe Ile Asn Trp Leu Ile Gln Thr Lys Ile Thr
            20                  25                  30

Asp

<210>  68
<211>  33
<212>  PRT
<213>  Artificial

<220>
<223>  2V-GLP-2

<400>  68

His Val Asp Gly Ser Phe Ser Asp Glu Met Asn Thr Ile Leu Asp Asn
1               5                   10                  15

Leu Ala Ala Arg Asp Phe Ile Asn Trp Leu Ile Gln Thr Lys Ile Thr
            20                  25                  30

Asp

<210>  69
<211>  29
<212>  PRT
<213>  Homo sapiens

<220>
<223>  Glucagon

<400>  69

His Ser Gln Gly Thr Phe Thr Ser Asp Tyr Ser Lys Tyr Leu Asp Ser
1               5                   10                  15

Arg Arg Ala Gln Asp Phe Val Gln Trp Leu Met Asn Thr
            20                  25

<210>  70
<211>  29
<212>  PRT
<213>  Artificial

<220>
<223>  12R-Glucagon

<400>  70

His Ser Gln Gly Thr Phe Thr Ser Asp Tyr Ser Arg Tyr Leu Asp Ser
1               5                   10                  15

Arg Arg Ala Gln Asp Phe Val Gln Trp Leu Met Asn Thr
            20                  25

<210>  71
<211>  29
<212>  PRT
<213>  Artificial

<220>
<223>  12R,20K-Glucagon

<400>  71

His Ser Gln Gly Thr Phe Thr Ser Asp Tyr Ser Arg Tyr Leu Asp Ser
1               5                   10                  15

Arg Arg Ala Lys Asp Phe Val Gln Trp Leu Met Asn Thr
            20                  25

<210>   72
<211>   29
<212>   PRT
<213>   Artificial

<220>
<223>   12R,24K-Glucagon

<400>   72

His Ser Gln Gly Thr Phe Thr Ser Asp Tyr Ser Arg Tyr Leu Asp Ser
1               5                   10                  15

Arg Arg Ala Gln Asp Phe Val Lys Trp Leu Met Asn Thr
            20                  25

<210>   73
<211>   29
<212>   PRT
<213>   Artificial

<220>
<223>   12R,29K-Glucagon

<400>   73

His Ser Gln Gly Thr Phe Thr Ser Asp Tyr Ser Arg Tyr Leu Asp Ser
1               5                   10                  15

Arg Arg Ala Gln Asp Phe Val Gln Trp Leu Met Asn Lys
            20                  25

<210>   74
<211>   29
<212>   PRT

<213>   Artificial

<220>
<223>   9E-Glucagon

<400>   74

His Ser Gln Gly Thr Phe Thr Ser Glu Tyr Ser Lys Tyr Leu Asp Ser
1               5                   10                  15

Arg Arg Ala Gln Asp Phe Val Gln Trp Leu Met Asn Thr
            20                  25

<210>   75
<211>   29
<212>   PRT
<213>   Artificial

<220>
<223>   9E,16E,29K-Glucagon

<400>   75

His Ser Gln Gly Thr Phe Thr Ser Glu Tyr Ser Lys Tyr Leu Asp Glu
1               5                   10                  15

Arg Arg Ala Gln Asp Phe Val Gln Trp Leu Met Asn Lys
            20                  25

<210>   76
<211>   29
<212>   PRT
<213>   Artificial

<220>
<223>   13K,17E-Glucagon

<400>   76

His Ser Gln Gly Thr Phe Thr Ser Asp Tyr Ser Lys Lys Leu Asp Ser
1               5                   10                  15

Glu Arg Ala Gln Asp Phe Val Gln Trp Leu Met Asn Thr

20                    25


<210>  77
<211>  29
<212>  PRT
<213>  Artificial

<220>
<223>  20E,24K-Glucagon

<400>  77

His Ser Gln Gly Thr Phe Thr Ser Asp Tyr Ser Lys Tyr Leu Asp Ser
1               5                   10                  15


Arg Arg Ala Glu Asp Phe Val Lys Trp Leu Met Asn Thr
            20                  25


<210>  78
<211>  9
<212>  PRT
<213>  Artificial

<220>
<223>  GIP(1-9) Analogs


<220>
<221>  misc_feature
<222>  (1)..(1)
<223>  Xaa is Tyr, D-Tyr, N-Acetyl-Tyr, N-Pyroglutamyl-Tyr,
 N-Glucitol-Tyr, N-Palmitate-Tyr, N-Fmoc-Tyr, N-alkyl-Tyr,
 N-glycosyl-Tyr, N-isopropyl-Tyr, or deletion

<220>
<221>  misc_feature
<222>  (2)..(2)
<223>  Xaa is Ala, D-Ala, Gly, Ser, 2-Aminobutylic acid,
 Aminoisobutylic acid, Phospho-serine, Sarcosine or deletion

<220>
<221>  misc_feature
<222>  (3)..(3)
<223>  Xaa is Glu, D-Glu, Pro, Hydroxyproline, Lys, Tyr or Phe

<220>
<221>  misc_feature
<222>  (9)..(9)
<223>  Xaa is Asp or Deletion

<400>  78

Xaa Xaa Xaa Gly Thr Phe Ile Ser Xaa
1               5

<210>  79
<211>  8
<212>  PRT
<213>  Artificial

<220>
<223>  2G-GIP(1-8)

<400>  79

Tyr Gly Glu Gly Thr Phe Ile Ser
1               5

<210>  80
<211>  8
<212>  PRT
<213>  Artificial

<220>
<223>  D2A-GIP(1-8)

<220>
<221>  misc_feature
<222>  (2)..(2)
<223>  Xaa is D-Ala

<400>  80

Tyr Xaa Glu Gly Thr Phe Ile Ser
1               5

<210>  81
<211>  8
<212>  PRT

<213>   Artificial

<220>
<223>   2Abu-GIP(1-8)

<220>
<221>   misc_feature
<222>   (2)..(2)
<223>   Xaa is Abu

<400>   81

Tyr Xaa Glu Gly Thr Phe Ile Ser
1               5

<210>   82
<211>   8
<212>   PRT
<213>   Artificial

<220>
<223>   2Aib-GIP(1-8)

<220>
<221>   misc_feature
<222>   (2)..(2)
<223>   Xaa is Aib

<400>   82

Tyr Xaa Glu Gly Thr Phe Ile Ser
1               5

<210>   83
<211>   8
<212>   PRT
<213>   Artificial

<220>
<223>   2PS-GIP(1-8)

<220>
<221>   misc_feature

<222>   (2)..(2)
<223>   Xaa is Phosphoserine

<400>   83

Tyr Xaa Glu Gly Thr Phe Ile Ser
1               5

<210>   84
<211>   8
<212>   PRT
<213>   Artificial

<220>
<223>   2Sar-GIP(1-8)

<220>
<221>   misc_feature
<222>   (2)..(2)
<223>   Xaa is MeGly (sarcosine)

<400>   84

Tyr Xaa Glu Gly Thr Phe Ile Ser
1               5

<210>   85
<211>   8
<212>   PRT
<213>   Artificial

<220>
<223>   3P-GIP(1-8)

<400>   85

Tyr Ala Pro Gly Thr Phe Ile Ser
1               5

<210>   86
<211>   8
<212>   PRT
<213>   Artificial

<220>
<223>   3Hyp-GIP(1-8)

<220>
<221>   misc_feature
<222>   (3)..(3)
<223>   Xaa is Hyp

<400>   86

Tyr Ala Xaa Gly Thr Phe Ile Ser
1               5

<210>   87
<211>   8
<212>   PRT
<213>   Artificial

<220>
<223>   3K-GIP(1-8)

<400>   87

Tyr Ala Lys Gly Thr Phe Ile Ser
1               5

<210>   88
<211>   8
<212>   PRT
<213>   Artificial

<220>
<223>   3Y-GIP(1-8)

<400>   88

Tyr Ala Tyr Gly Thr Phe Ile Ser
1               5

<210>   89
<211>   8
<212>   PRT
<213>   Artificial

<220>
<223>   3F-GIP(1-8)

<400>   89

Tyr Ala Phe Gly Thr Phe Ile Ser
1               5

<210>   90
<211>   9
<212>   PRT
<213>   Artificial

<220>
<223>   2G-GIP(1-9)

<400>   90

Tyr Gly Glu Gly Thr Phe Ile Ser Asp
1               5

<210>   91
<211>   9
<212>   PRT
<213>   Artificial

<220>
<223>   2S-GIP(1-9)

<400>   91

Tyr Ser Glu Gly Thr Phe Ile Ser Asp
1               5

<210>   92
<211>   9
<212>   PRT
<213>   Artificial

<220>
<223>   D2A-GIP(1-9)

<220>
<221>   misc_feature

<222>   (2)..(2)
<223>   Xaa is D-Ala

<400>   92

Tyr Xaa Glu Gly Thr Phe Ile Ser Asp
1               5

<210>   93
<211>   9
<212>   PRT
<213>   Artificial

<220>
<223>   2Abu-GIP(1-9)

<220>
<221>   misc_feature
<222>   (2)..(2)
<223>   Xaa is Abu

<400>   93

Tyr Xaa Glu Gly Thr Phe Ile Ser Asp
1               5

<210>   94
<211>   9
<212>   PRT
<213>   Artificial

<220>
<223>   2Aib-GIP(1-9)

<220>
<221>   misc_feature
<222>   (2)..(2)
<223>   Xaa is Aib

<400>   94

Tyr Xaa Glu Gly Thr Phe Ile Ser Asp
1               5

<210>  95
<211>  9
<212>  PRT
<213>  Artificial

<220>
<223>  2PS-GIP(1-9)

<220>
<221>  misc_feature
<222>  (2)..(2)
<223>  Xaa is Phosphoserine

<400>  95

Tyr Xaa Glu Gly Thr Phe Ile Ser Asp
1               5

<210>  96
<211>  9
<212>  PRT
<213>  Artificial

<220>
<223>  2Sar-GIP(1-9)

<220>
<221>  misc_feature
<222>  (2)..(2)
<223>  Xaa is MeGly (sarcosine)

<400>  96

Tyr Xaa Glu Gly Thr Phe Ile Ser Asp
1               5

<210>  97
<211>  9
<212>  PRT
<213>  Artificial

<220>
<223>  3P-GIP(1-9)

<400>   97

Tyr Ala Pro Gly Thr Phe Ile Ser Asp
1               5

<210>   98
<211>   9
<212>   PRT
<213>   Artificial

<220>
<223>   3Hyp-GIP(1-9)

<220>
<221>   misc_feature
<222>   (3)..(3)
<223>   Xaa is Hyp

<400>   98

Tyr Ala Xaa Gly Thr Phe Ile Ser Asp
1               5

<210>   99
<211>   9
<212>   PRT
<213>   Artificial

<220>
<223>   3K-GIP(1-9)

<400>   99

Tyr Ala Lys Gly Thr Phe Ile Ser Asp
1               5

<210>   100
<211>   9
<212>   PRT
<213>   Artificial

<220>
<223>   3Y-GIP(1-9)

<400>   100

Tyr Ala Tyr Gly Thr Phe Ile Ser Asp
1               5



<210>   101
<211>   9
<212>   PRT
<213>   Artificial

<220>
<223>   3F-GIP(1-9)

<400>   101

Tyr Ala Phe Gly Thr Phe Ile Ser Asp
1               5



<210>   102
<211>   9
<212>   PRT
<213>   Artificial

<220>
<223>   GLP-1(7-15) Analogs



<220>
<221>   misc_feature
<222>   (2)..(2)
<223>   Xaa is Ala, Gly, Ser, Thr, Leu, Ile, Val, Glu, Lys or Aib

<220>
<221>   misc_feature
<222>   (3)..(3)
<223>   Xaa is Glu, Gly or Lys

<220>
<221>   misc_feature
<222>   (9)..(9)
<223>   Xaa is Asp or deletion

<400>   102

His Xaa Xaa Gly Thr Phe Thr Ser Xaa

1               5

<210>  103
<211>  8
<212>  PRT
<213>  Artificial

<220>
<223>  2G-GLP-1(7-14)

<400>  103

His Gly Glu Gly Thr Phe Thr Ser
1               5

<210>  104
<211>  8
<212>  PRT
<213>  Artificial

<220>
<223>  2V-GLP-1(7-14)

<400>  104

His Val Glu Gly Thr Phe Thr Ser
1               5

<210>  105
<211>  8
<212>  PRT
<213>  Artificial

<220>
<223>  2Aib-GLP-1(7-14)

<220>
<221>  misc_feature
<222>  (2)..(2)
<223>  Xaa is Aib

<400>  105

His Xaa Glu Gly Thr Phe Thr Ser

1                    5

<210>   106
<211>   9
<212>   PRT
<213>   Artificial

<220>
<223>   2S-GLP-1(7-15)

<400>   106

His Ser Glu Gly Thr Phe Thr Ser Asp
1                    5

<210>   107
<211>   9
<212>   PRT
<213>   Artificial

<220>
<223>   2G-GLP-1(7-15)

<400>   107

His Gly Glu Gly Thr Phe Thr Ser Asp
1                    5

<210>   108
<211>   9
<212>   PRT
<213>   Artificial

<220>
<223>   2V-GLP-1(7-15)

<400>   108

His Val Glu Gly Thr Phe Thr Ser Asp
1                    5

<210>   109
<211>   9
<212>   PRT

<213>   Artificial

<220>
<223>   2Aib-GLP-1(7-15)

<220>
<221>   misc_feature
<222>   (2)..(2)
<223>   Xaa is Aib

<400>   109

His Xaa Glu Gly Thr Phe Thr Ser Asp
1               5

<210>   110
<211>   7
<212>   PRT
<213>   Artificial

<220>
<223>   GLP-2(1-7)

<400>   110

His Ala Asp Gly Ser Phe Ser
1               5

<210>   111
<211>   8
<212>   PRT
<213>   Artificial

<220>
<223>   GLP-2(1-8)

<400>   111

His Ala Asp Gly Ser Phe Ser Asp
1               5

<210>   112
<211>   16
<212>   PRT

<213>   Artificial

<220>
<223>   GIP(1-16)

<400>   112

Tyr Ala Glu Gly Thr Phe Ile Ser Asp Tyr Ser Ile Ala Met Asp Lys
1               5                   10                  15


<210>   113
<211>   42
<212>   PRT
<213>   Artificial

<220>
<223>   2S-GIP

<400>   113

Tyr Ser Glu Gly Thr Phe Ile Ser Asp Tyr Ser Ile Ala Met Asp Lys
1               5                   10                  15


Ile His Gln Gln Asp Phe Val Asn Trp Leu Leu Ala Gln Lys Gly Lys
            20                  25                  30


Lys Asn Asp Trp Lys His Asn Ile Thr Gln
        35                  40